# EUROPEAN PATENT APPLICATION

(11) **EP 4 822 711 A2**
(43) Date of publication of application: **14.10.2026**
(21) Application number: 26185835.1
(22) Date of filing: 10.07.2019
(51) Int. Cl.: G16H 50/30

(54) **HEALTHCARE MANAGEMENT METHOD**

(30) Priority: 11.07.2018 JP 2018131293
(62) Document number(s) of the earlier application(s) in accordance with Art. 76 EPC: 19834564.7 / 3 822 639
(71) Applicant: Provigate Inc., Tokyo 113-0023 (JP)
(72) Inventor: SEKIMIZU, Koshin, Tokyo, 113-0033 (JP); ITO, Narushi, Tokyo, 113-0033 (JP); KATAYAMA, Norikazu, Tokyo, 113-0033 (JP)
(74) Representative: Michalski Hüttermann & Partner mbB

(57) **Abstract**

The present disclosure provides a healthcare management method comprising acquiring a first data that includes a glycoalbumin concentration and an albumin concentration in a body fluid from a subject; generating, on the basis of the first data, output information related to the GA value; and providing the generated output information to a user.

## Description

The present disclosure relates to a healthcare management method.

A plurality of markers are used as indices indicating the control state of the blood glucose level. For example, in hospitals, glycated hemoglobin (HbA1c) has been measured for diabetic patients. Glycated hemoglobin (HbA1c) is an indicator of the glycation rate of red blood cell hemoglobin. The interval of hospital visits for diabetic patients is usually 1 to 3 months. Because the half-life of red blood cells is about 36 days, this marker is an indicator of blood glucose levels over a period of 1 to 2 months. HbA1c levels are measured once every 1 to 3 months for diabetic patients and once a year for healthy people, such as health examinations. Next, glycated albumin (glycoalbumin, GA), which is a blood glucose control index, is a marker indicating the glycation degree of albumin in blood. Albumin has a half-life of about 17 days, indicating a blood glucose status of about 2 weeks. In addition, 1,5-anhydro-D-glucitol (1,5-AG) is a marker for blood glucose levels over several days.

In the present disclosure, there are provided a method of healthcare management related to a blood glucose level, a method of providing healthcare information, a method of diabetes risk management, a countermeasure method of diabetic patient complication, and the like. In a further embodiment, first data comprising a glycated albumin concentration and an albumin concentration in a body fluid of a subject (tears, saliva, blood, and the like.) may be acquired. In a further embodiment, output information associated with the GA level may be generated based on the first data. In a further embodiment, the generated output information may be provided to a user.
FIG. 1 shows the relationship between blood GA levels and tear GA levels.
FIG. 2 shows the relationship between blood GA levels and saliva GA levels.
FIG. 3 is a flowchart of a method of health management or the like, according to an embodiment of the present disclosure.
FIG. 4 is a flowchart of a method of health management or the like, according to an embodiment of the present disclosure.
FIG. 5 is a graph showing weekly changes in 75th percentiles of blood glucose levels and blood GA levels.
FIG. 6 is a graph showing weekly changes in 90th percentiles of blood glucose levels and blood GA levels.
FIG. 7 is a graph showing weekly changes in median blood glucose levels and blood GA levels.
FIG. 8 shows the relationship between 75th percentiles of blood glucose levels and blood GA levels taken weekly for each subject.
FIG. 9 shows the relationship between 75th percentiles of blood glucose levels and normalized blood GA levels taken weekly.
FIG. 10 is a diagram showing a typical example of a normal dietary life period.
FIG. 11 is a diagram showing a typical example of a hypoglycemic phase.
FIG. 12 shows the relationship between 75th percentiles of blood glucose levels and blood GA levels taken weekly in two subjects.
FIG. 13 shows the relationship between blood GA levels corrected by uric acid level and 75th percentiles of blood glucose levels.
FIG. 14 shows the relationship between the total area within the 25th to 75th percentiles of blood glucose levels and the blood GA levels in two subjects.
FIG. 15 shows a comparison of the blood GA levels corrected by uric acid level and the total area within 25th-75th percentiles of blood glucose levels.
FIG. 16 is a graph showing weekly changes in body weight and blood GA levels.
FIG. 17 is a graph showing weekly changes in body weight and 75th percentiles of blood glucose levels.
FIG. 18 is a block diagram of a health management system according to an embodiment of the present disclosure.
FIG. 19 is a block diagram of a GA measurement device according to an embodiment of the present disclosure.
FIG. 20 schematically shows a configuration of first data.

Unless otherwise defined, all technical and scientific terms used herein have the same meaning as commonly understood by those skilled in the art to which this invention belongs.

Where a range of values is provided, unless the context clearly dictates otherwise, one tenth of the units of the upper and lower limits of the range and intervening values within the indicated range or any other recited value are encompassed within the scope of the present invention. The upper and lower limits of smaller ranges may be independently included in a smaller range and are also included within the scope of the present invention, provided that any specifically excluded limitation within the described range. If the described ranges include one or both of the limits, ranges excluding any of their included limits are also included in the present invention. Of course, it is not explicitly stated that the term "about" precedes all numerical designations. All numerical representations, including ranges, e.g., length, volume, pH, temperature, time, concentration, and molecular weight, are approximations that vary from a numerical value of the smallest order of magnitude to an upper (+) or lower (-). The term "about" also includes the exact value "X" in addition to a small increment of "X", such as "X+0.1" or "X-0.1". The reagents described herein are merely illustrative, and equivalents thereof are known in the art.

In the following description, numerous specific details are set forth to provide a more complete understanding of the invention. However, it will be apparent to those skilled in the art that the present invention may be practiced with one or more of these specific details. In other instances, features and procedures well known to those skilled in the art have not been described to avoid obscuring the present invention.

As used herein, the terms "including" and "comprising" are intended to mean that the configurations and methods include the recited elements but do not exclude others. Embodiments defined by each of these transition terms are within the scope of the invention. Thus, the methods and compositions, configurations can "include" additional steps and components.

The present disclosure provides, without limitation, methods healthcare management related to GA levels or blood glucose levels, healthcare information provision, diabetes risk management, diabetes patient complication management, diabetes diagnosis, and programs for performing the methods, storage media for storing the programs, and data structures related thereto.

The present disclosure includes, but is not limited to, the following:
Diagnostic methods for diabetes based on GA levels. First data including GA levels in body fluids, such as tears, saliva, blood, and the like, used for diabetes diagnosis. The use of first data comprising GA levels in body fluids for the diagnosis of diabetes. For diagnosing diabetes mellitus, a comprehensive judgment may be made by referring to the second data such as blood glucose levels and HbA1c, diabetes type, symptoms, symptoms of diabetic retinopathy, the content of repeated examinations, and the content of the physician's medical record.

Albumin is produced primarily in the liver and supplied systemically via the blood. The albumin is secreted from the glands as other body fluids. Albumin, for example, is secreted from the blood via lacrimal glands and salivary glands and is also present in tears and saliva.

The inventors have found that the GA level representing the degree of glycation of albumin in body fluids, i.e., glycated albumin concentration/ albumin concentration, is correlated with the GA level in blood. The correlation between the GA levels of tears and blood and the correlation between the GA levels of saliva and blood, which were found, are shown below.

### EXAMPLE 1

### <Comparison of Tear GA Level and Blood GA Level>

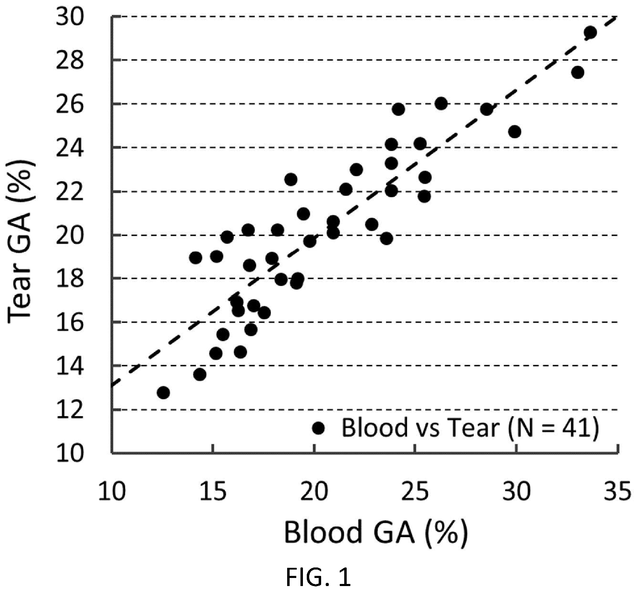

The GA level of tears (tear GA level) and the GA level of blood (blood GA level) were measured. As a sample solution, tears and blood collected from 41 diabetic volunteers with informed consent were used. The GA level in blood was measured by an enzymatic method using a commercially available kit, Lucica (registered trademark) GA-L (Asahi Kasei Pharma Co., Ltd.). As for tear fluid, the collected tear fluid was first subjected to reduction and alkylation treatment, and then it was subjected to trypsin digestion at 37°C overnight. After that treatment, the GA level in the tears was measured by liquid chromatography mass spectrometry (LC-MS/MS). FIG. 1 shows a plot of blood GA levels and tear GA levels. One point indicates the value of each sample.

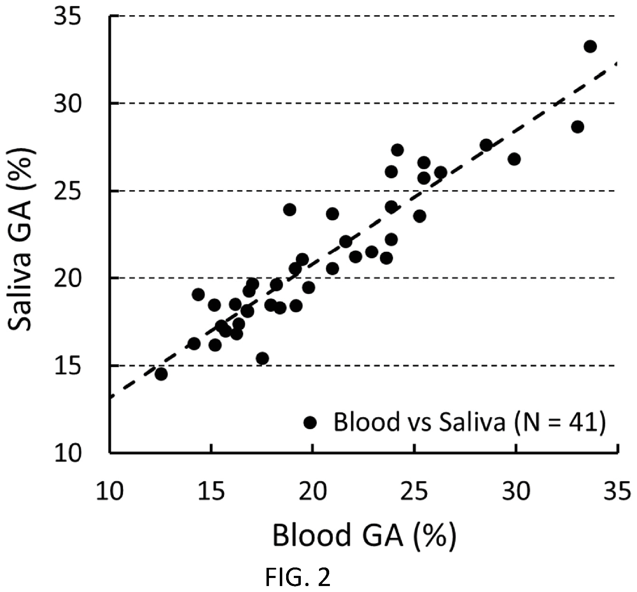

The inventors compared the enzymatic and LC-MS/MS assays as methods for obtaining the blood GA level, and identified a very strong positive correlation between them (not shown). Thus, the plots in FIGS. 1 and 2 use blood GA levels determined by enzymatic assays and tear GA and salivary GA levels determined by LC-MS/MS assays, respectively.

FIG. 1 is a graph showing the relationship between blood GA levels (X-axis) and tear GA levels (Y-axis). The correlation was found to have a correlation coefficient r=0.891, p<0.01. Thus, the inventors have determined that there is a very strong positive correlation between tear GA levels and blood GA levels. The results suggest that the tear GA level may be a biomarker showing a mean blood glucose level for 1 to 2 weeks as an alternative of the blood GA level.

### EXAMPLE 2

### <Comparison of Saliva GA Level and Blood GA Level>

GA levels in the saliva (tear GA levels) and GA levels in the blood (blood GA levels) were measured. As a sample solution, tears and blood collected from 41 diabetic volunteers who obtained the same informed consent as in Example 1 were used. The GA level in the blood was measured by an enzyme method using a commercially available kit, Lucica GA-L (Asahi Kasei Pharma Corporation). Regarding saliva, the collected saliva was first subjected to reduction and alkylation treatment, and then it was subjected to trypsin digestion at 37°C overnight. After the treatment, GA levels in saliva were measured by liquid chromatography mass spectrometry (LC-MS/MS). FIG. 2 shows a plot of blood GA levels and salivary GA levels. One point indicates the value of each sample.

FIG. 2 is a graph showing the relationship between the blood GA levels (X-axis) and salivary GA levels (Y-axis). The correlation was found to have a correlation coefficient r=0.927 and p<0.01. Thus, the inventors have determined that there is a very strong positive correlation between salivary GA levels and blood GA levels. The results suggest that the salivary GA level may be a biomarker showing a mean blood glucose level for 1 to 2 weeks as an alternative to the blood GA level.

All of the embodiments, aspects, examples, numerical values, and the like shown based on the blood GA levels in the present disclosure can be replaced with GA levels obtained for body fluids (including, for example, tears, tears). Even if only the blood GA level is described in the present disclosure, embodiments and the like which are read into the GA level of a body fluid (e.g., including tears and tears) are naturally included in the present disclosure.

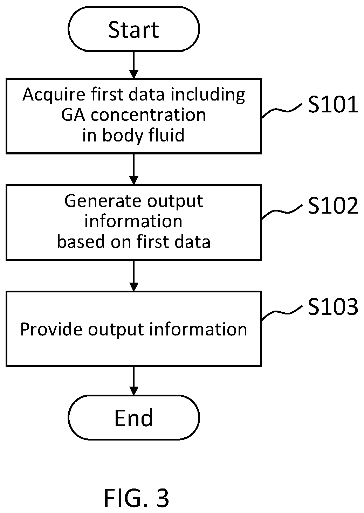

According to one embodiment of the present disclosure shown in FIG. 3, a healthcare management method, in step S101, acquires or collects the first data containing the glycated albumin levels in the body fluid of the subject, in step S102, generates output information associated with the GA levels, based on the first data, and in step S103, provides the generated output information to the user.

The "subject" may be a human or a terrestrial animal. The terrestrial animal may be a mammal. The terrestrial animal may be a pet animal, a livestock, a domestic animal, or a wild animal. The subject may be a subject of one body (one person, one, one animal, or the like), may be a plurality of subjects, may be a subject of at least one body, and may be one or a plurality of subjects.

The sample to be tested may be a solution. The solution may be a body fluid, a solution derived from a body fluid, and may be a dilute fluid of a body fluid. The solution may be a solution that is not a body fluid (derived from a non-body fluid), and may be a mixture of a body fluid or a solution derived from a body fluid and a solution derived from a non-body fluid. The solution may be a solution used for sample measurement and may be a solution used for measurement for calibration. For example, the solution may be a standard solution or a calibration solution.

The "body fluid" may be blood, serum, plasma, lymph fluid, tissue fluids such as interstitial fluid, transcellular fluid, interstitial fluid, and the like, and may be body cavity fluid, serosal fluid, pleural fluid, ascites fluid, pericardial fluid, cerebrospinal fluid, joint fluid (synovial fluid), and aqueous humor of the eye (aqueous). The body fluid may be digestive fluid such as saliva, gastric juice, bile, pancreatic juice, intestinal fluid, and the like, and may be sweat, tears, nasal mucus, urine, semen, vaginal fluid, amniotic fluid, milk, or the like. The body fluid may be a body fluid of an animal and may be a body fluid of a human. The "body fluid" may be a liquid (milk, dairy, etc.) in a food product containing a protein derived from an animal. The body fluid may be a body fluid of a plant, a plant biological fluid, or a liquid derived from a plant. For example, the body fluid may be fruit juice, dense, sap of a plant. The "body fluid" may be a solution.

In some embodiments, the solution may comprise a physiological buffer. The solution may include an object to be measured. The buffer may be a buffer called Good's buffers (Good buffers). The buffer may include phosphate buffered saline (PBS) or N-tris(hydroxymethyl)methyl-2-aminoethanesulfonic acid buffer (TES). The buffers may include at least one of or a mixture of 2-morpholinoethanesulfonic acid (MES), 3-morpholinopropane sulfonic acid (MOPS), 2-hydroxy-3-morpholinopropane sulfoic acid (MOPSO), piperazine-1,4-bis(2-hydroxy-3-propane sulfonic acid) dihydrate (POPSO), N-(2-acetamide)iminodiacetic acid (ADA), piperazine-1,4-bis(2-ethanesulfonate)sodium (PIPES), N-(2-acetamide)-2-aminoethansulfonic acid (ACES), cholamine chloride hydrochloride, N,N-bis(2-hydroxyethyl)-2-aminoethanesulfonic acid (BES), N-tris(hydroxymethyl)methyl-2-aminoethanesulfonic acid (TES), 4-(2-hydroxyethyl)-1-piperazine ethanesulfonic acid (HEPES), Sodium2-[4-(2-hydroxyethyl)-1-piperazinyl]ethanesulfonate (HEPES-Na), acetamidoglycine, tricine, glycinamide, bicine, bis(2-hydroxyethyl)iminotris(hydroxymethylmethane) (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethane sulfonic acid (CHES), 3-[N,N-bis(2-hydroxyethyl)amino]-2-hydroxypropane sulfonic acid (DIPSO), 3-[4-(2-hydroxyethyl)-1-piperadinyl]propane sulfonic acid (HEPPS), 2-hydroxy-3-[4-(2-hydroxyethyl)-1-piperadinyl]propanesulfonic acid monohydrate (HEPPSO), N-tris(hydroxymethyl)methyl-3-aminopropanesulfonic acid (TAPS), and 2-hydroxy-N-tris(hydroxymethyl)methyl-3-aminopropanesulfonic acid (TAPSO).

Acquiring or collecting a "glycated albumin concentration" or "albumin concentration" may be acquiring or collecting an output of a sensor, a "glycated albumin concentration" or "albumin concentration" itself, or a value associated therewith, or the like. Collecting "glycated albumin concentration" or "albumin concentration" may include measuring the respective concentration. Acquisition or collection may include obtaining an albumin concentration and a glycated albumin concentration by measurement in a body fluid of a subject. Acquisition or collection may include acquiring or collecting first data comprising albumin concentration and glycated albumin concentration.

In some embodiments, acquiring or collecting may include time information of measurement of albumin concentration and glycated albumin concentration or time or related time information of collection of a body fluid (tears, saliva, blood, etc.) to be measured. In some embodiments, acquiring or collecting may include collecting first data including albumin concentration and glycated albumin concentration and time information of measurement thereof. In some embodiments, it may be acquired as time information regarding secretion, collection or measurement of a body fluid of a subject to be measured. In some embodiments, the time of measurement may be recorded by a sensor or a measurement device as time information. In a case where there is a time from collection to measurement, the time of collection, the time of measurement, or both may be acquired as time information.

"Acquiring or collecting" may include collecting body fluid from a subject. In one aspect, tears or saliva may be collected from a subject. In one aspect, blood may be collected from a subject. In some embodiments, the sample may be collected non-invasively. In some embodiments, the sample may be collected invasively.

Tear as a sample may be taken from the surface of the subject's eye, the eyelid, the cornea, the conjunctiva, or the vicinity thereof. The tear may be collected from the lacrimal tract, such as the punctum, canaliculus, lacrimal sac, and the like, or may be collected from the nose. Saliva as a sample may be taken from saliva or slaver of the subject, and may be taken from the oral cavity. Blood may be collected by conventional blood collection methods.

In some embodiments, body fluid may be collected from a subject and immediately subjected to a measurement. For example, a sensor or the like may be brought into close proximity to the eye or the like, and a measurement may be performed instantaneously. In some embodiments, it may be collected once in a container or sensor, and thereafter subjected to a measurement. In some embodiments, a measurement may be conducted some time after collection. In some embodiments, the body fluid may be stored after collection until a measurement. The storage method may be cryopreservation. For example, body fluid may be collected over multiple times, stored for a period of time, and collectively measured.

In some embodiments, measurements of the albumin concentration and the glycated albumin concentration may be performed on body fluids (tears, saliva, blood, and the like) collected at substantially the same time or secreted or collected at substantially the same time. In some embodiments, a measurement may be performed simultaneously or at another timing with collection of the sample. In some embodiments, a measurement may be performed using the same sensor or multiple sensors. The albumin concentration and the glycated albumin concentration may be measured at different timings, and may be measured in a body fluid (tear fluid, or saliva, blood, or the like) collected or secreted at different timings, or may be measured in different body fluids.

"Glycated albumin concentration" or "albumin concentration" is the concentration of glycated albumin or albumin, respectively, in a sample (tears or saliva) taken or of interest. When a sensor is used, an output (current, voltage, optical signal, etc.) corresponding to or related to a "glycated albumin concentration" or an "albumin concentration" may be obtained, and each concentration may be calculated from the output based on the association. The data to be acquired or collected may directly be a "glycated albumin concentration" or an "albumin concentration" or the output value of the sensor corresponding thereto, or may be some value, signal, information, data corresponding to a "glycated albumin concentration" or an "albumin concentration" based on the value in the middle of the conversion, i.e. the output of the sensor.

Methods for measuring GA levels include enzymatic methods, high-performance liquid chromatography, mass spectrometry, and isotope dilution mass spectrometry, and the like. As clinical examinations enzymes are mainly used. In the enzymatic methods, glycated albumin is decomposed into amino acids by protease, of which ketoamine oxidase which reacts only with glycated amino acids is acted, and H₂O₂ generated by the action is measured to measure the glycated albumin concentration. Albumin may be reacted to a bromocresol purple (BCP) reagent to measure albumin concentration from the absorbance change of the blue conjugate. The GA level can be calculated as [glycated albumin concentration/albumin concentration] × 100 (%)

In some embodiments, a glycated protein sensor comprising an immobilized protease, an immobilized ketoamine oxidase and a hydrogen peroxide detector may be used to measure the concentration of glycated albumin.

By using such a sensor, for example, the concentration of glycated albumin in tears or saliva can be measured non-invasively. Thus, GA levels can be measured without stress on the subject, either physically or psychologically, or with minimum of such stress.

In some embodiments, acquiring or collecting an albumin concentration and a glycated albumin concentration of a body fluid (tear, or saliva, blood, etc.) may include acquiring or collecting an albumin concentration and a glycated albumin concentration in a body fluid multiple times. In some embodiments, acquiring or collecting may include collecting albumin concentrations and glycated albumin concentrations in a plurality of body fluids in a time series. In some embodiments, a data array having time or time information corresponding to measurements of glycated albumin concentration may be generated.

In some embodiments, acquiring or collecting an albumin concentration and a glycated albumin concentration in a body fluid multiple times may be collecting the glycated albumin amount in tears 2 times.

In some embodiments, acquiring or collecting an albumin concentration and a glycated albumin concentration in a plurality of body fluids may be collecting an albumin concentration and a glycated albumin concentration in tears 3 or more times. In doing so, the amount of glycated albumin in the tear may be collected three or more times at a certain or varying time intervals. When collecting a plurality of albumin concentrations and glycated albumin concentrations, the interval between measurements or the interval at which tears to be measured are collected may be constant or variable. The time interval between acquisitions or collections of albumin concentrations and glycated albumin concentrations may vary from time to time, and only a portion thereof may vary if there are a few intervals.

The frequency of acquiring, collecting or measuring samples may be substantially 1 time/month, 2 times/month or higher than their values. The frequency may be less than or equal to 20 times/month, 10 times/month, 8 times/month, 1 time/month, or the like. The frequency may be substantially 2 times/month, 3 times/month, 4 times/month, or 0.5 times/week, 1 time/week.

The frequency of acquisition, collection, or measurement may be substantially once every 17 days in some embodiments, and may be as low as once every 2 weeks in some embodiments.

The values of the frequency of acquisition, collection or measurement may be converted into collection intervals and used.

Acquisition and collection (hereinafter referred to as "collection") periods, or collection frequency, or periods covered used to calculate collection characteristics may be 2 weeks, 3 weeks, 4 weeks, 5 weeks, 6 weeks, 7 weeks, 8 weeks, 9 weeks, 10 weeks, 11 weeks, 12 weeks, 1 month, 2 months, 3 months, or the like.

Collections or measurements may be taken at an average of 7 days, an average of 10 days, an average of 14 days or more. Collections or measurements may be taken at an average of 21 days, an average of 24 days, an average of 28 days or less.

In some embodiments, the first data may be collected by a computer, or a computer system. In some embodiments, the collected location data may be input to a computer, a computer system, a memory, or a storage medium, and may be further saved or stored therein.

The output information is generated based on the first data. In some embodiments, output information may be generated by a computer or a computer system, based on the first data.

In some embodiments, output information is associated with a GA level. In some embodiments, output information may be a GA level. Generally, the GA level can be calculated as glycated albumin concentration (mg/dL)/albumin concentration (mg/dL). $GA level \left(%\right) = glycated albumin \left(mg/dL\right) / albumin concentration \left(mg/dL\right) \times 100$

The GA level obtained from a concentration of glycated albumin in tears or saliva may be referred to as tear GA level or saliva GA level. The GA level obtained from a concentration of glycated albumin in a non-invasively collected body fluid may be referred to as non-invasive GA level. The GA level obtained from a concentration of glycated albumin in the blood may be referred to as blood GA level. The GA level obtained from a concentration of glycated albumin in an invasively collected body fluid may be referred to as invasive GA level.

"Output information" generated and provided to a user may be the GA level at each measurement, may be the most recent GA level, may be a trend of past GA levels, or may be one or more GA levels.

Further, the output information related to the GA level includes, but is not limited to, the following.
• Difference from previous value = previous GA level -current GA level

| | |
|---|---|
| • | Converted HbA1c level (%) = GA level × 0.245 + 1.73 (another conversion formula may also be used.) |
| • | Estimated mean blood glucose level (HbA1c conversion: average over the past 36 days) = 28.7 × converted HbA1c - 46.7 |

• Estimated mean blood glucose level (GA conversion: average over the past 17 days) is a data table of GA levels and mean blood glucose levels.

The half-life of GA level is said to be 17 days. The actual interval of samplings or measurements of samples may not be exactly the half-life. In some embodiments, a deviation between the measurement interval and the half-life may be considered or corrected, to calculate the mean blood glucose level. In some embodiments, the mean blood glucose level corresponding to the half-life may be determined from a measurement interval using its decay curve (negative exponential curve).

In some embodiments, a mean GA level or a blood glucose level in the period from (n-1)-th measurement to n-th measurement may be obtained by conversion. In some embodiments, a mean GA level or a blood glucose level of a corresponding period may be obtained by conversion from the GA levels obtained from two consecutive measurements. In some embodiments, a mean GA level or a blood glucose level for the relevant period may be obtained from GA levels obtained from multiple measurements. In some embodiments, a mean GA level or a blood glucose level may be obtained by dividing the sum of GA levels obtained from multiple measurements by the number of times. In some embodiments, the mean blood glucose level may be obtained as a weighted function of the ratio obtained at each time, and the weighting may be obtained, as an example, as a larger set function of the ratio obtained at a newer measurement time.

Generating output information may use data other than the first data (second data or the secondary data). The second data includes data relating to non-subjects. In some embodiments, the second data may be a collection of data (individual-specific data) related to individual non-subjects. In some embodiments, the second data may be data extracted or processed from data related to a plurality of subjects. In some embodiments, the second data may be data (population-specific data) including trends, properties, characteristics, and the like for a particular population of subjects.

The second data may be classified, by way of example but not limitation, into data groups such as personal data, health-related data, lifestyle habit data, population data, and the like. The data groups of the second data and the second data items for each are listed below by way of example:
- Personal data: personal ID ("my number"), age, gender, marital status, number of children, address, residence type, owned cars, owned motorcycles, owned bicycles, job, job type, job position, annual salary, genomic information, belief, health insurance number, fingerprint, voice print, face recognition information, iris recognition information, time-changing personal ideas, emotion, desire/request, input to smart devices and usage information thereof, and the like.
- Population data: race, nationality, region, district area, seasonal variation factors (such as seasonal temperature and humidity changes), holiday periods (including year-end and spring holidays, summer holidays, etc.), means of commutation, religion, and the like. • Health-related data: body weight, height, body fat, maximal blood pressure, minimal blood pressure, heart rate, step count, activity, blood oxygenation, body temperature, room temperature, solar radiation, UV radiation, mean blood glucose levels obtained from continuous blood glucose level measurements (Continuous Glucose Monitoring (CGM), Flash Glucose Monitoring (FGM)) devices, standard deviations, Ambulatory Glucose Profile (AGP), health checkup data (may include HbA1c), medical history, prescription medications, medication information, data from health institutions, food and other allergies, family history and health-related data, and the like.
- Lifestyle habit data: dietary habits (including carnivores, vegetarians, and the like.), tastes, meal menus, meal photographs, snacks, exercise habits, wake-up time, bedtime, sleeping time, sleep depth, sleep points, active or inactive rest times, meal speed, chewing frequency/rate/characteristics, food allergies, religious faith-related dietary rules (including Ramadan, and the like.), exercise status, exercise items, exercise time, exercise frequency, exercise consumption calories, hobbies, and the like.

The items of the second data, the definition of data groups, the distribution of each second data item to the data groups, and the like are not limited to the above. In some embodiments, a computer or the like, artificial intelligence, or the like, may be used to create a data group, such as by characteristics found in the existing second data, or to classify the existing second data into a new data group. In some embodiments, information or data about correlations or associations within existing second data may be classified, stored, or utilized as second data.

In some embodiments, the second data may include time-related information. In some embodiments, the second data may include information regarding the order of eating during a meal, exercise, and timing of meals. In some embodiments, the second data may include timing of taking the medication. In some embodiments, depending on the efficacy of insulin, such as its immediate and delayed efficacy, the timing of insulin administration, as well as the timing and time-related information of meals and exercises may be included.

In some embodiments, the second data may be acquired from a subject, a collector, or a user. In some embodiments, it may be acquired from other individuals, organizations, or the like. In some embodiments, the second data provider may be asked for an indication of whether there is an opt-in (permission to use the personal data). In some embodiments, the second data may be acquired and collected from a subject, a collector, or a user, or a person associated therewith, using an application.

In some embodiments, a subject, a user or other individual, enterprise organization, or the like may be prompted to provide the second data or information related to the second data, or receive inquiries, questions, or the like.

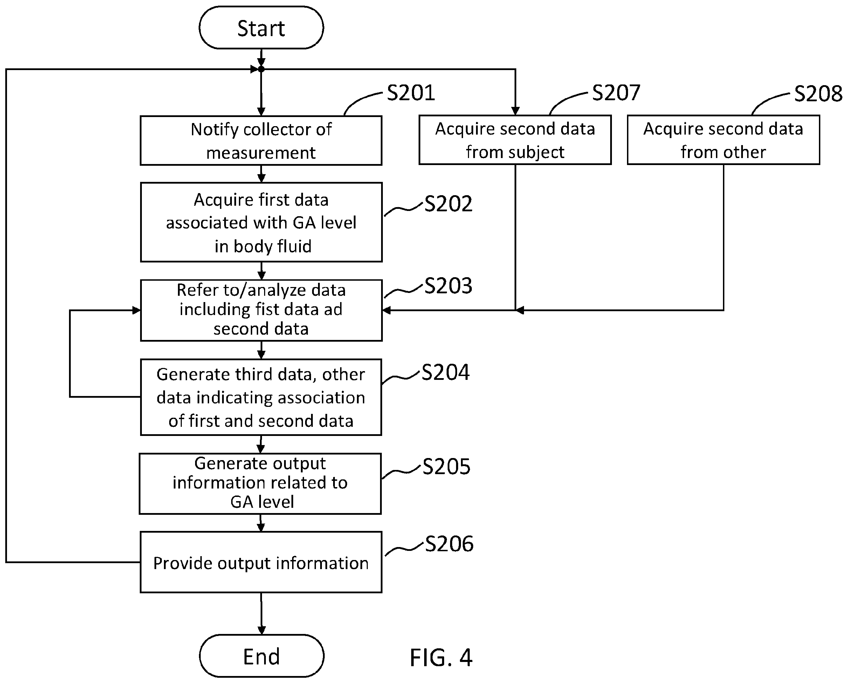

FIG. 4 illustrates, in some embodiments, a method of using the first data as well as the second data, to generate and provide output information regarding the GA level. In some embodiments, these steps are performed using a computer system.

First, in step S201, the collector is notified that a body fluid of the subject is to be sampled and measured.

Next, in step S202, first data on GA levels in the collected body fluids are acquired or collected. For example, the concentration of glycated albumin (GA) in the body fluid is measured and its concentration is collected as first data. In some embodiments, the concentration of albumin in the same body fluid may be measured. The GA level can be obtained from the ratio between the concentration of glycated albumin and the concentration of albumin.

In step S203, the collected first data and second data are referred to, and these data are analyzed. In some embodiments, other data may be used in addition to the first data and the second data.

In step S207, second data on the subject may be acquired from the subject. That is, data relating to the subject may be handled as second data. Alternatively, second data may be acquired from other than a subject, i.e. from a non-subject, in step S208.

Subsequently, in step S204, the association or correlation between the first data and the second data is searched from the analysis in step S203. In some embodiments, if some association is found, it may be generated and stored as third data (tertiary data). In some embodiments, even though no obvious association is found, if an event is found that suggests a possible association, or even though no association is found, or if any data is generated, it may be stored as other data. These data can also be returned to step S203 to repeat further analysis, if useful or as a routine.

Next, in step S205, the output information to be provided to the user is generated.

Then, in step S206, the output information is provided to the user. If the user is a collector, it is provided to the collector. The provision of the output information to the collector may be performed at the same time as the notification that the measurement is to be performed to the collector, may be performed at a time associated with the timing of the notification, or may be performed at a timing unrelated to the notification.

In some embodiments, information on the control state of the blood glucose level during or in connection with the GA measurement may be included in the second data, and analysis using artificial intelligence including machine learning and deep learning may be performed. In some embodiments, an analysis may predict the risk of developing future diabetes or its complications, and other diseases.

In some embodiments, among the second data, the health data of the subject may be used to make a false diagnostic check or determination as to whether the GA level obtained in the measurement is a false high value or a false low value. In some embodiments, alert information may be provided to the user based on these false diagnosis checks or determinations. Several examples are described below.

### • GA False High Level Check

In some embodiments, when the second data includes information on liver cirrhosis, hypothyroidism, and administration of a thyroid hormone synthesis inhibitor, attention that the acquired GA level is likely to be a false high level may be generated as output information. The GA level acquired by a measurement may be higher than the true value due to the effect of a disease, administration or taking of a therapeutic agent. For example, protein synthesis is slowed in the cases of cirrhosis, hypothyroidism, or in the cases of a thyroid hormone synthesis inhibitor (Mercazole (registered trademark) or the like) being taken. That is, the production and metabolism of albumin are delayed, and the half-life of albumin is extended. As a result, the GA level is increased.

### • GA False Low Level Check

In some embodiments, when the second data includes information on nephrotic syndrome, protein-leaking gastroenteropathy, hyperthyroidism, and thyroid hormone therapeutics, attention that the acquired GA level is likely to be a false low level may be generated as output information. The GA level acquired by a measurement may be lower than the true value due to the effect of a disease, administration or taking of a therapeutic agent.

Examples include nephrotic syndrome, protein-leaking gastroenteropathy, and the like. In such cases, protein excretion out of the body leads to increased biosynthesis to compensate for albumin deficiency, resulting in a shortened albumin half-life. That is, the denominator of the GA level becomes high and the GA level becomes low. Also, for example, in the case of hyperthyroidism or in the case of taking thyroid hormone (Thyradine S (registered trademark) or the like), the half-life of albumin is shortened because of increased metabolism. As a result, the GA level is decreased.

### • GA-Converted HbA1c Level Check

In some embodiments, when the second data includes HbA1c levels measured at a medical institution, or the like, they are compared with the GA-converted HbA1c levels of the most recent first data, and when the measured HbA1c level ≥ the GA-converted HbA1c level, output information may be generated that the mean blood glucose level is improved. In some embodiments, when the second data includes HbA1c levels measured at a medical institution, or the like, they are compared with the GA-converted HbA1c levels of the most recent first data, and when the measured HbA1c level < the GA-converted HbA1c level, output information may be generated that the mean blood glucose level is deteriorated.
- Check and Correlation Between GA Levels and Continuous Glucose Level Measurements In some embodiments, the data of continuous blood glucose measurement devices included in the second data may be compared to the measured GA levels to generate a correlation therebetween. In some embodiments, the continuous blood glucose measurement may comprise a method of estimating a blood glucose trend from a glucose concentration (glucose level) in blood or interstitial fluid measured continuously for several days. In some embodiments, the second data may include Ambulatory Glucose Profile (AGP) based on continuous blood glucose measurements. In some embodiments, a correlation or conversion formula between the measured GA level and the mean glucose level by CGM may be obtained. The data of CGM obtained by measurements of the number of days over a certain measurement period may be graphed as a one-day profile from 0 o'clock to 24 o'clock in the form of superposition for that number of days. In some embodiments, variation data or a statistical value (mean value, variation range, standard deviation (SD), or the like) of glucose levels obtained from sustained glucose measurements performed over a period of time, for example 2 weeks, may be obtained. The variation range may be a value of deviation from a mean value (or a statistical value or a mathematical value indicating a median or other center) of glucose level at each time point in data superimposed as a one-day profile, for example. The variation range may be the maximum and/or minimum values, such as from the mean value of glucose levels at each time point, or a value of 10-90% (10-90th percentile), a value of 25-75% (25-75th percentile) of the total variation, or the standard deviation (SD). In some embodiments, the relationship between the most recently measured GA level or the measured GA level at a point in time associated with the duration of the continuous blood glucose measurement may be determined.

### EXAMPLE 3

### <Comparison of Blood Glucose Level and GA Level>

The relationship between the blood glucose level and the blood GA level was examined. Four healthy volunteers from whom informed consent was obtained were attached with a FreeStyle Libre (registered trademark, Abbott Japan Co., Ltd.), and the interstitial liquid glucose level was continuously measured for three months. The FreeStyle Libre has been re-attached about every two weeks. It is known that the absolute value of the measured values of FreeStyle Libre varies up and down with respect to the true value. Therefore, in such cases, blood glucose levels were measured by SMBG using Glutest ai (registered trademark, Sanwa Chemical Laboratory Co., Ltd., Arkray Factory Co., Ltd.) at the same time as Libre measurements at a certain frequency during the period of attached measurements. The blood glucose level in the interstitial fluid is corrected on the basis of the blood glucose level measured by SMBG, and converted to the blood glucose level (also referred to as CGM-corrected blood glucose level). Blood was collected every week, to measure blood GA levels.

During the continuous monitoring period, healthy volunteers were asked to cooperate so as to live with normal diet, hypoglycemic diet, and normal diet again each for 4 weeks for the period of 3 months of measurements, to collect CGM-corrected blood glucose levels.

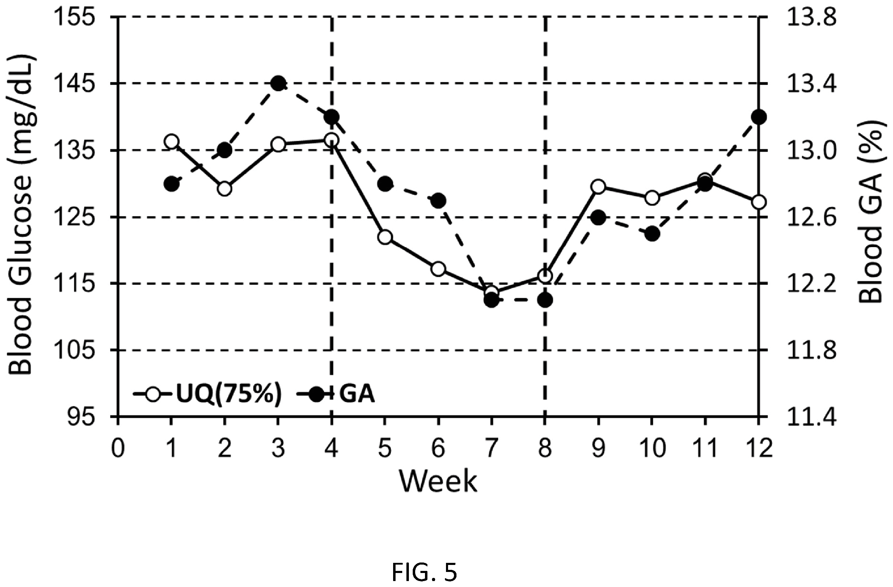

FIG. 5 shows the change in blood GA level and 75th percentile of the CGM-corrected blood glucose level, taken weekly from one healthy volunteer. The changes in blood GA level and the changes in 75th percentile of blood glucose level are quite consistent, indicating a very strong positive correlation between them (correlation coefficient r=0.749). A strong positive correlation was also confirmed in other healthy volunteers.

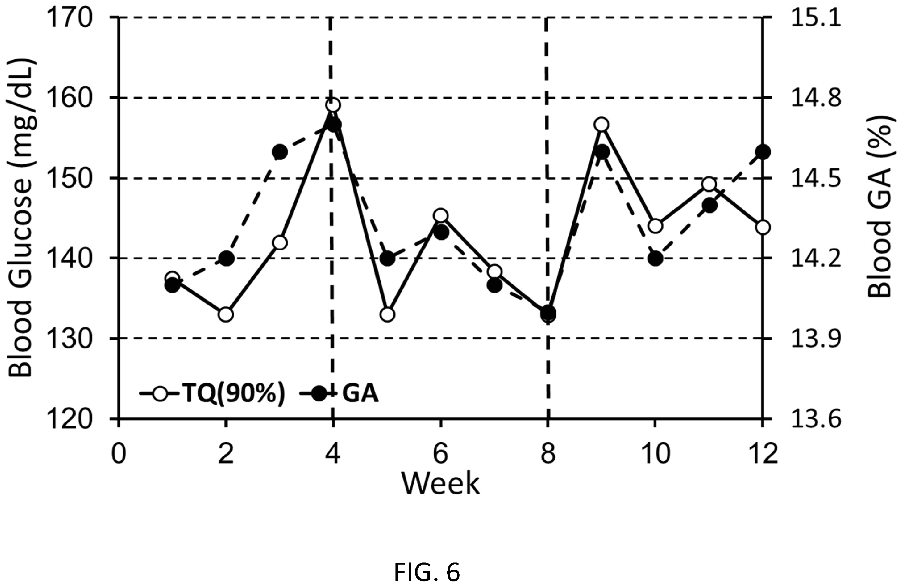

FIG. 6 shows the weekly changes in blood GA level and 90th percentile of the CGM corrected blood glucose level. Similar to the results of the 75th percentile blood glucose level, the changes in blood GA level and 90th percentile of blood glucose level for a 3-month period are quite consistent, with a very strong positive correlation between them (correlation coefficient r=0.786).

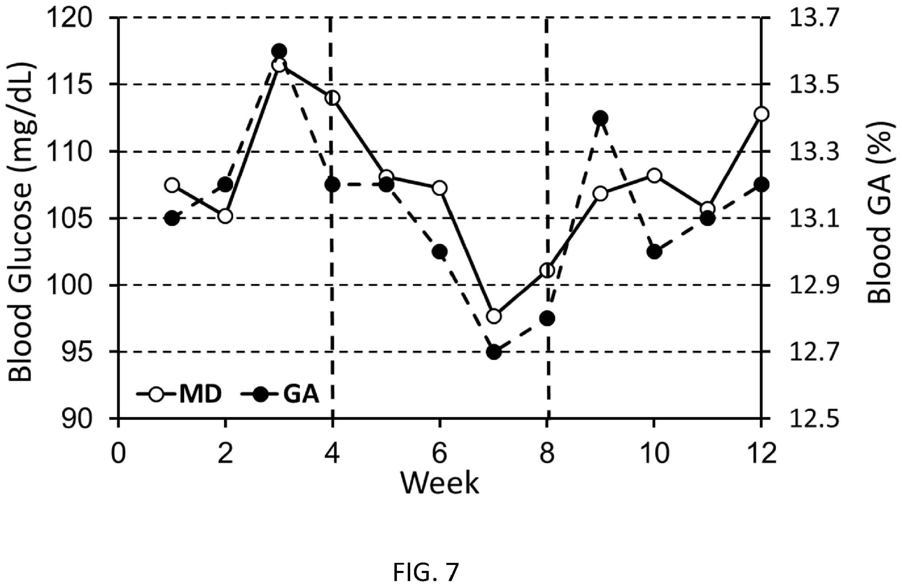

FIG. 7 shows the weekly changes in blood GA level and median value of the CGM corrected blood glucose level. Similar to the results of the 75th percentile of blood glucose level and and 90th percentile of blood glucose level, it has become clear that there is a very strong positive correlation between the blood GA level and the median of the blood glucose level (correlation coefficient r=0.801).

As can be understood from the above results, the weekly changes in blood glucose level can be inferred from the weekly blood GA levels without continuous monitoring using CGM or the like.

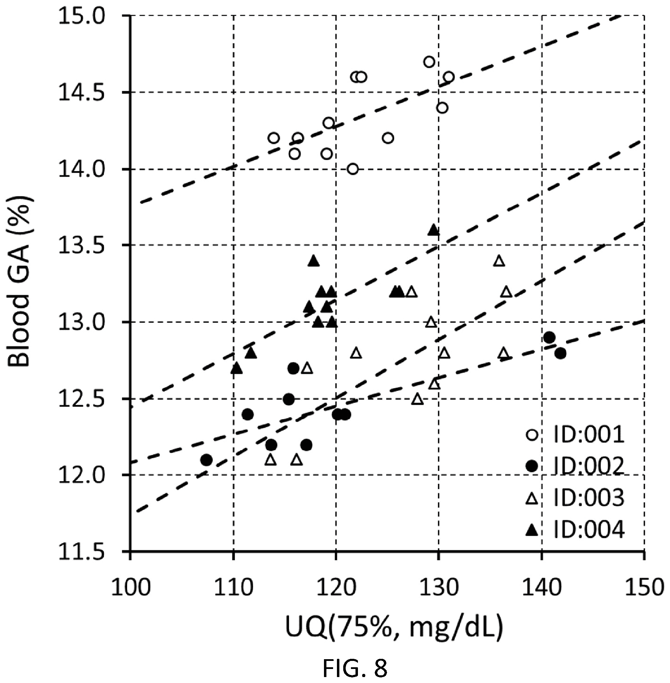

FIG. 8 shows a plot of weekly blood GA levels and 75th percentile of CGM corrected glucose levels for each subject, based on data obtained over 12 weeks for four healthy volunteers (ID 001 to ID 004).

The relationship between the weekly blood GA levels and the 75th percentile values has been found to vary from subject to subject. In some embodiments, the coefficients of the correlation equation between the blood GA levels and the 75th percentiles (for example, a and b at y=ax+b, if approximated by a linear function, or the like.) may be set for each subject. In some aspects, it may be set for each subject by referring to some or all of the information included in the secondary data.

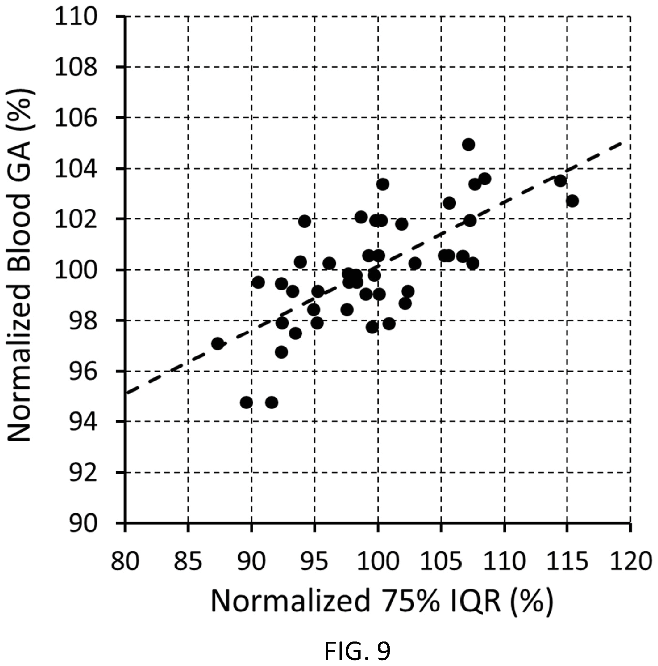

FIG. 9 shows a plot of weekly blood GA levels and 75th percentiles of CGM corrected glucose for four healthy volunteers divided by 12-week averages and converted (normalized) to relative values for each subject. The correlation was found to have a correlation coefficient r=0.711, p<0.01. That is, the inventors have determined that there is a very strong positive correlation between the variation in blood GA level and the variation in 75th percentile, by relativization or normalization.

In some embodiments, a 75th percentile of CGM or a value associated therewith may be estimated or calculated, based on the GA level over a period of time (for example, one week).

### EXAMPLE 4

### <Comparison of Postprandial Hyperglycemia Peak Frequency and Blood GA Level>

Similar to Example 3, blood GA levels and CGM corrected blood glucose levels were collected over a three-month period from four healthy volunteers with informed consent.

The blood GA levels were compared with the number of blood glucose levels over a threshold per day over a weekly transition. Table 1 shows the correlation coefficient between the blood GA level obtained from each healthy volunteer (ID:0001 to ID:0004) and the number of times the CGM-corrected blood glucose level exceeds each threshold value, for each threshold value (unit mg/dL).

**Table 1**

| ID | ≧80 | ≧90 | ≧100 | ≧110 | ≧120 | ≧130 | ≧140 | ≧150 | ≧160 | ≧170 | ≧180 |
|---|---|---|---|---|---|---|---|---|---|---|---|
| 001 | 0.152 | 0.268 | 0.226 | 0.481 | 0.628 | 0.738 | 0.742 | 0.793 | 0.781 | 0.796 | 0.711 |
| 002 | 0.791 | 0.750 | 0.727 | 0.763 | 0.745 | 0.773 | 0.817 | 0.808 | 0.784 | 0.762 | 0.750 |
| 003 | 0.641 | 0.818 | 0.688 | 0.691 | 0.700 | 0.747 | 0.730 | 0.674 | 0.564 | 0.607 | 0.649 |
| 004 | 0.670 | 0.660 | 0.684 | 0.796 | 0.759 | 0.675 | 0.624 | 0.389 | 0.229 | 0.209 | -0.218 |

Table showing the correlation coefficients when comparing the number of occurrences of blood glucose above the threshold value per day with the blood GA level.

For the healthy volunteer with ID:001, the correlation coefficient exceeded 0.481 when the blood glucose level was 110 mg/dL or higher, and exceeded 0.628 when the blood glucose level was 120 mg/dL or higher, indicating a strong positive correlation. At glucose levels of 130mg/dL or higher, the correlation coefficient exceeds 0.738, indicating a stronger positive correlation (Table 1).

For the healthy volunteer with ID:004, the correlation exceeded 0.600 from the case the blood glucose level was 80 mg/dL or higher to the case the blood glucose level was 140 mg/dL or higher, indicating a strong positive correlation. On the other hand, the correlation coefficient decreases at the blood glucose levels of 150 mg/dL or higher, suggesting that the positive correlation is weak (Table 1).

Thus, the relationship between the value of the hyperglycemic peak and the number or frequency of occurrence may vary from individual to individual. In some embodiments, for each individual, the blood glucose range of the hyperglycemic peaks (a postprandial hyperglycemic peak, for example without limitation) may be examined by measurement, or may be appropriately set. In some embodiments, data of the relationship between the frequency of hyperglycemic peaks and the GA levels may be accumulated. In one aspect, the GA level after one week may be predicted from data over a time range of less than one week, for example three days. In some embodiments, the frequency of hyperglycemia (for example, postprandial hyperglycemia peaks) may be predicted from measured GA levels. In one aspect, health forecast may be generated based on them and may provide advice on diet, eating habits, and lifestyle habit. Analysis related to the above may be performed using AI.

### EXAMPLE 5

### <Comparison of Blood Glucose Level and GA Level 3>

Similar to Example 3, blood GA levels and CGM corrected blood glucose levels were collected over a three-month period from four healthy volunteers with informed consent.

Table 2 shows the correlation coefficient between the blood GA level obtained from each healthy volunteer (ID:001 to ID:004), the CGM-corrected blood glucose level for one week, and the cumulative area (unit [mg/dL] *time) of the portion exceeding each threshold value based on the data calculated from all data, for each threshold value.

**Table 2**

| **ID** | **≧50** | **≧70** | **≧80** | **≧90** | **≧100** | **≧110** | **≧120** | **≧130** | **≧140** | **≧150** | **≧160** | **≧170** |
|---|---|---|---|---|---|---|---|---|---|---|---|---|
| **001** | **0.423** | **0.422** | **0.430** | **0.450** | **0.468** | **0.496** | **0.673** | **0.802** | **0.796** | **0.767** | **0.725** | **0.670** |
| **002** | **0.773** | **0.773** | **0.772** | **0.768** | **0.766** | **0.769** | **0.774** | **0.779** | **0.780** | **0.767** | **0.740** | **0.694** |
| **003** | **0.823** | **0.808** | **0.797** | **0.780** | **0.760** | **0.754** | **0.756** | **0.742** | **0.705** | **0.667** | **0.661** | **0.648** |
| **004** | **0.754** | **0.754** | **0.753** | **0.749** | **0.747** | **0.732** | **0.671** | **0.553** | **0.366** | **0.204** | **0.014** | **-0.096** |

Table showing the correlation coefficient when comparing the area under the curve of blood glucose above the threshold value per day with the blood GA level.

For the healthy volunteer with ID:001, the correlation coefficient exceeded 0.400 when the blood glucose level was 50 mg/dL or higher, was almost 0.500 when the blood glucose level was 110 mg/dL or higher, and exceeded 0.600 when the blood glucose level was 120 mg/dL or higher. A strong positive correlation was observed even at the threshold values higher than those. Thus, it has become clear that the cumulative area of blood glucose level was strongly positively correlated with the blood GA level at or above any threshold (Table 2).

For the healthy volunteer with ID:004, the correlation coefficient exceeded 0.750 when the blood glucose level was 50 mg/dL or higher, and exceeded 0.700 when the blood glucose level was 110 mg/dL or higher. The correlation coefficient exceeded 0.500 even when the blood glucose level was 130 mg/dL or higher. A strong positive correlation was observed at these thresholds. On the other hand, when the threshold value was 140 mg/dL or higher, no strong positive correlation was observed (Table 2).

Thus, the relationship between the blood GA level and the cumulative area of blood glucose levels per day can vary from individual to individual. In some embodiments, for each individual, the blood glucose range of the hyperglycemic peak (a postprandial hyperglycemic peak, for example without limitation) may be examined by measurements, or may be appropriately set. In some embodiments, data of the relationship between the cumulative area and the GA level may be accumulated. In one aspect, the GA level after one week may be predicted from data associated with a cumulative area of less than one week, for example, three days. In some embodiments, the cumulative area of the blood glucose level may be predicted from measured GA levels. In one aspect, health forecast may be generated based on them and may provide advice on diet, eating habits, and lifestyle habit. Analysis related to the above may be performed using AI.

### EXAMPLE 6

### <Comparison Between Blood Glucose Level AGP and Blood GA Level>

Similar to Example 3, blood GA levels and CGM corrected blood glucose levels were collected over a three-month period from four healthy volunteers with informed consent.

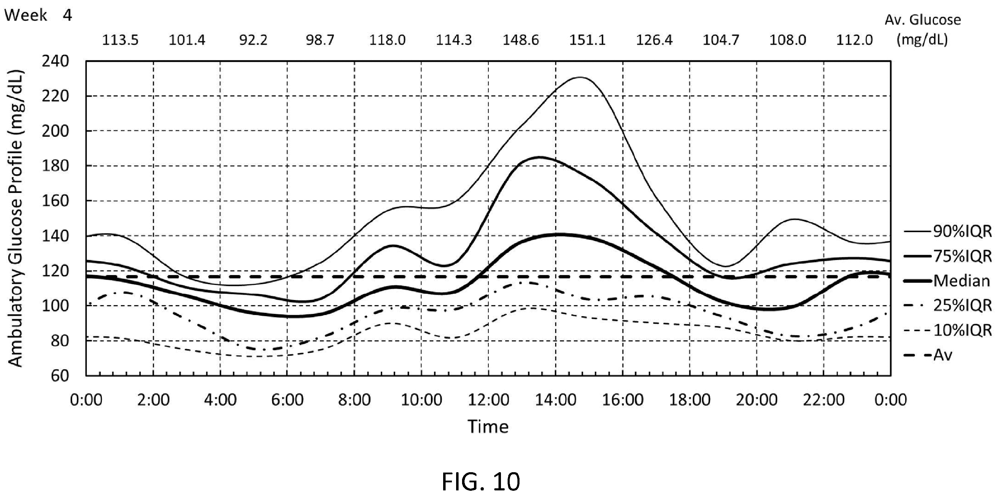

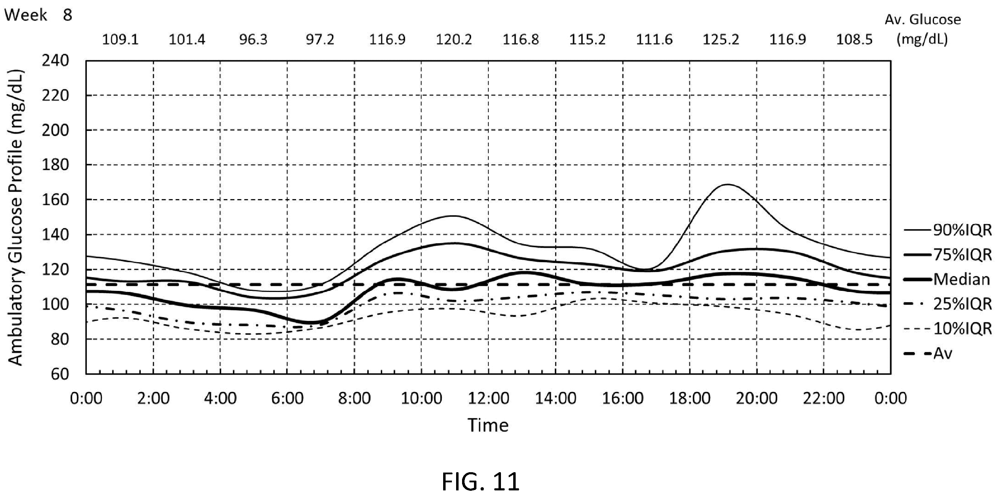

AGPs were generated using weekly continuously monitored blood glucose levels. FIG. 10 shows a representative example of AGP in the normal eating period, and FIG. 11 shows a representative example of AGP in the low-sugar eating period, respectively. The inner area (area between curves) sandwiched between two lines in the graph was calculated. Table 3 shows the correlation coefficients between this area and the blood GA level. The weekly change in area within each percentile was found to be consistent well with the change in blood GA level in many places. For example and not by way of limitation, the area within 25th percentiles and 75th percentiles, the area within 10th percentiles and 75th percentiles, the area within 2th percentiles and 75th percentiles, or the like, have been found to be strongly positively correlated for all four healthy volunteers.

**Table 3**

| **ID** | **10-25%** | **10-50%** | **10-75%** | **10-90%** |
|---|---|---|---|---|
| **001** | **0.329** | **0.460** | **0.701** | **0.771** |
| **002** | **0.705** | **0.832** | **0.848** | **0.809** |
| **003** | **0.146** | **0.356** | **0.627** | **0.544** |
| **004** | **0.584** | **0.721** | **0.732** | **0.522** |

| | **ID** | **25-50%** | **25-75%** | **25-90%** |
|---|---|---|---|---|
| | **001** | **0.530** | **0.757** | **0.805** |
| | **002** | **0.840** | **0.844** | **0.805** |
| | **003** | **0.397** | **0.682** | **0.525** |
| | **004** | **0.785** | **0.761** | **0.389** |

| | | **ID** | **50-75%** | **50-90%** |
|---|---|---|---|---|
| | | **001** | **0.683** | **0.769** |
| | | **002** | **0.808** | **0.736** |
| | | **003** | **0.732** | **0.492** |
| | | **004** | **0.335** | **-0.107** |

| | | | **ID** | **75-90%** |
|---|---|---|---|---|
| | | | **001** | **0.723** |
| | | | **002** | **0.561** |
| | | | **003** | **0.073** |
| | | | **004** | **-0.367** |

Table showing correlation coefficients when comparing the area between curves of each percentile of blood glucose level and the blood GA level, based on AGP.

On the other hand, there was no strong positive correlation at certain points, such as the area within 2th percentiles and 10th percentiles, the area within 2th percentiles and 25th percentiles, and the area within 90th percentiles and 98th percentiles, and the like.

The change in area within each percentile produced when AGP was created suggests that it can be inferred from the weekly blood GA levels. In some embodiments, from blood GA, the area within each percentile that can be obtained when making either or both of AGPs before and after the blood collection may be inferred or calculated. In some aspects, the percentile or range thereof to be employed may be set for each individual.

### EXAMPLE 7

### <Comparison of Blood GA Level Corrected by Uric Acid Level and Blood Glucose Levels >

Similar to Example 3, blood GA levels and CGM corrected blood glucose levels were collected over a three-month period from four healthy volunteers with informed consent.

In some embodiments, the correlation between the GA levels and blood glucose levels can be enhanced by correction. In one aspect, blood GA levels can be corrected using uric acid level to enhance the correlation between GA levels and blood glucose levels.

### <Example: Correlation Between Blood GA levels Corrected by Uric Acid Level and 75th percentiles of CGM-Corrected Blood Glucose levels >

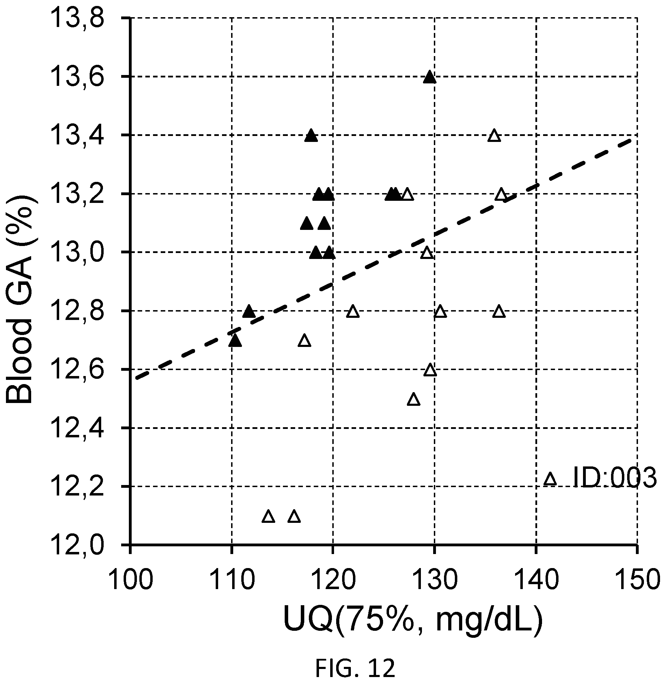

FIG. 12 shows the relationship between weekly blood GA levels and 75th percentiles of CGM corrected blood glucose levels for each healthy volunteer.

In the data shown in FIG. 12, between healthy volunteers, the correlation between the weekly variation of the blood GA level and the 75th percentiles of CGM corrected blood glucose levels has the correlation coefficient r=0.343, p=0.101.

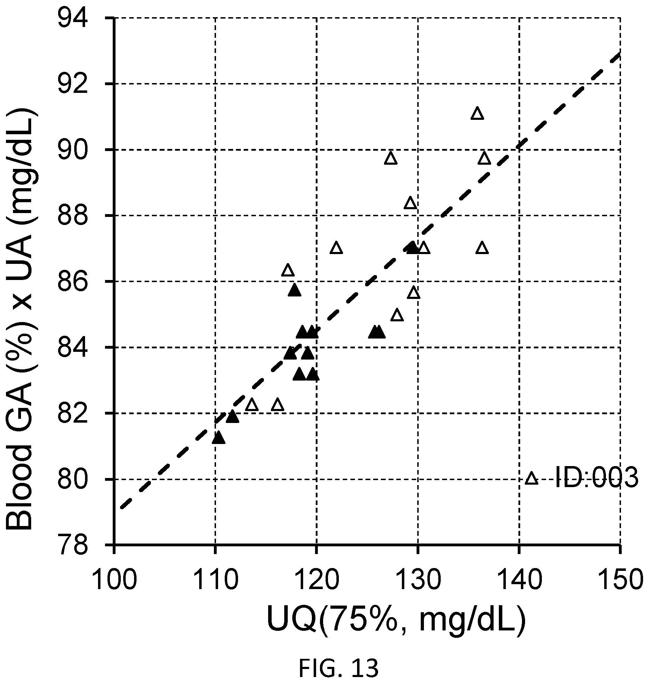

FIG. 13 shows the relationship between the blood GA level corrected by uric acid value level of each healthy volunteer and the 75th percentile of CGM corrected blood glucose levels. The correlation was found to have a correlation coefficient r=0.823, p<0.01. Thus, the inventors have determined that by correcting the GA level with the uric acid level, the correlation between the blood GA level and the CGM corrected blood glucose level becomes strong over a plurality of subjects.

### <Example: Correlation Between Blood GA levels Corrected by Uric Acid Level and Area From 25th to 75th percentiles>

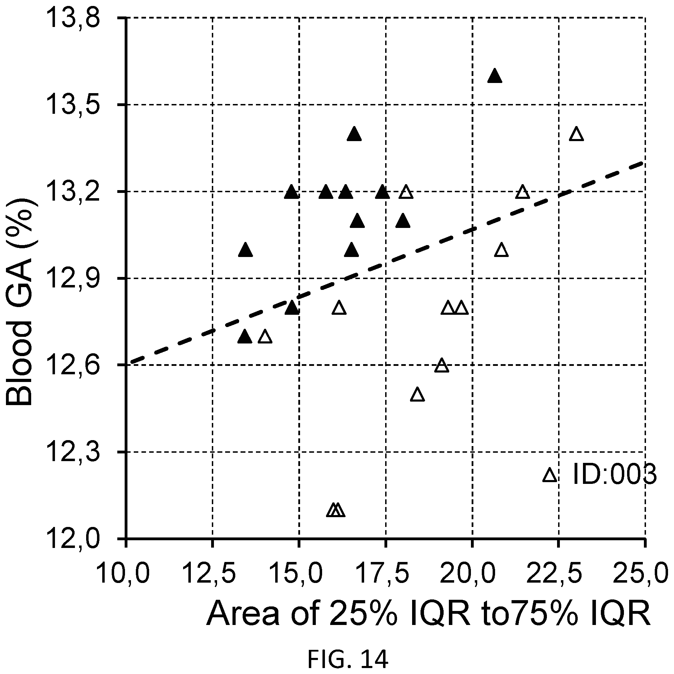

FIG. 14 shows the relationship between weekly blood GA level and the total inner area sandwiched between the 25th percentiles and the 75th percentiles of the CGM corrected blood glucose levels based on AGP for each healthy volunteer. The correlation was found to have a correlation coefficient r=0.319, p=0.129.

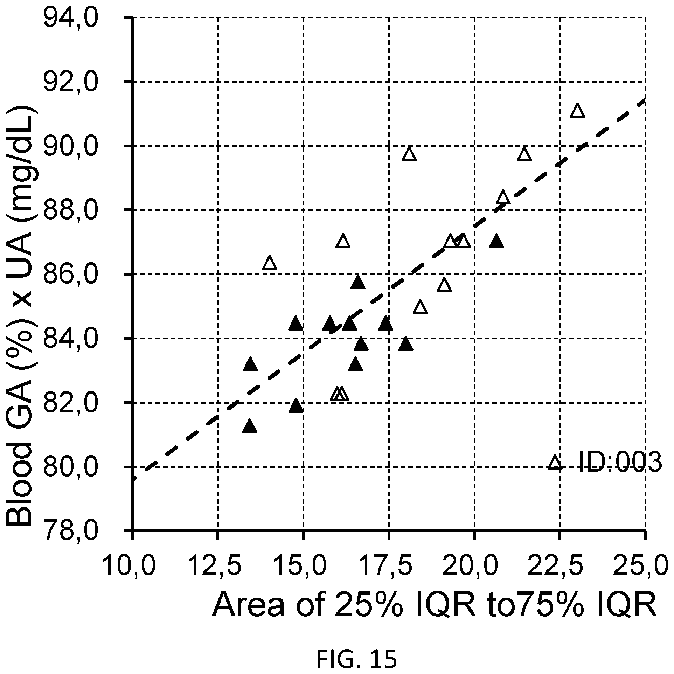

FIG. 15 shows the relationship between the weekly blood GA levels corrected by the uric acid level of each healthy volunteer and the total inner area sandwiched between the 25th percentiles and the 75th percentiles of CGM corrected blood glucose level based on the AGP. The correlation was found to have a correlation coefficient r=0.771, p<0.01. Thus, the inventors have determined that by correcting with the uric acid level, the positive correlation between the blood GA level and the cumulative area of the blood glucose level calculated from the AGP becomes strong over a plurality of subjects.

From the above results, it was found that the correlation between the variation in blood GA level and the variation in blood glucose value can be improved by correcting the blood GA level by uric acid level. In one aspect, the correction of the uric acid level may be a function of uric acid level. The function of correction by uric acid level may be set in accordance with whether or not the patient is diabetic, the type of diabetes mellitus, postprandial hyperglycemia, the manner of variation in blood glucose level, the state of renal function, the state of hepatic function, metabolic function, insulin resistance, obesity, or the like. In a further aspect, a correction may be made in which the blood GA level is multiplied by uric acid level to multiply by a coefficient. Uric acid levels may be used to correct for a selected population of subjects using a portion of certain secondary data. Corrections are not limited to the use of uric acid levels or uric acid values. Corrections may be made with other chemicals, biological substances, or values or functions associated therewith. In some embodiments, the correction can improve the correlation between the variation in blood GA levels and the variation in glucose level over a plurality of subjects. In some aspects, the correction may be made with different measurements performed on the same subject. In one aspect, the correction may be performed at a plurality of points or at a plurality of measurements performed at the same time. In one aspect, the correction may be made over a plurality of measurements made at different times. In one example, when measurement value on a day shows a result that deviates from this correlation, it may be determined that it suggests that the uric acid level may be abnormal. Physiological or mathematical values or functions, such as uric acid levels, may be used for correction.

### EXAMPLE 8

### <Comparison of Body Weight and Blood GA Level or Blood Glucose Level>

Similar to Example 3, blood GA levels and CGM corrected blood glucose levels were collected over a three-month period from four healthy volunteers with informed consent.

In some embodiments, the variation in body weight may be estimated from the GA level or the variation in GA level. In some embodiments, the relationship between the GA level and the body weight may be accumulated in the data. A time lag between the variation in GA level and the variation in body weight may be included to estimate the future body weight variation. In one aspect, the time lag may be stored as data. The relationship between the GA level or the variation in GA level and the variation in body weight may be set in accordance with the characteristics of the individual subject.

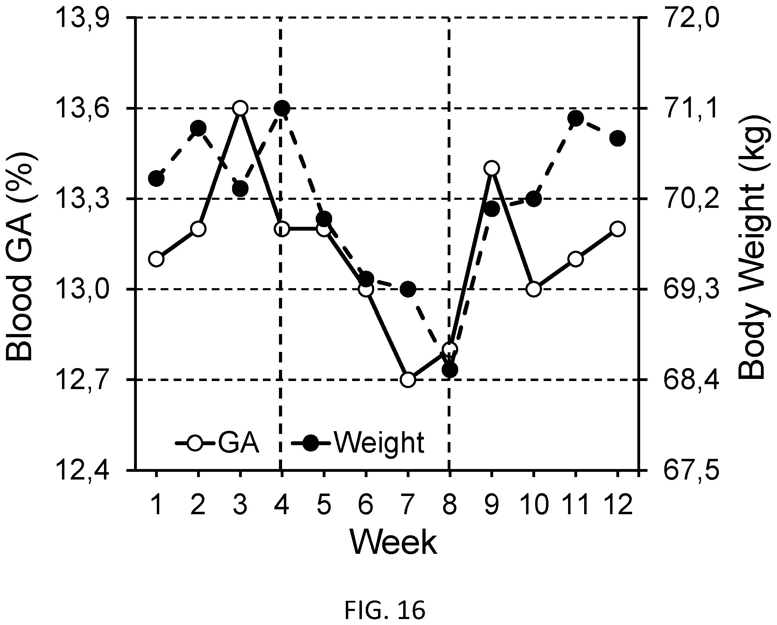

FIG. 16 shows the weekly changes of the blood GA level and the body weight obtained in one healthy volunteer. The change in blood GA levels and the change in body weight appeared to be slightly preceded by the blood GA levels, but there was a strong positive correlation between them (correlation coefficient r=0.568). A strong positive correlation was also observed in other healthy volunteers.

In some embodiments, the variation in body weight may be estimated from the variation in blood glucose level or statistics associated with the blood glucose levels. In some embodiments, the relationship between the blood glucose level or the like and the body weight may be accumulated in the data. Future body weight variation may be estimated, including the time lag between the variation in blood glucose level, or the like and the variation in body weight. In one aspect, the time lag may be stored as data. The relationship between the variation in blood glucose level or the like and the variation in body weight may be set in accordance with the characteristics of the individual subject.

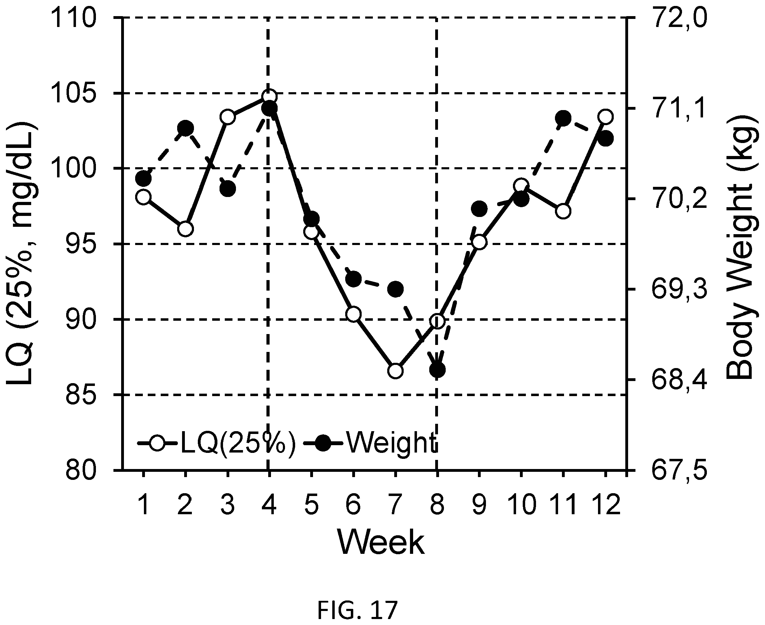

FIG. 17 shows weekly changes in 25th percentile of CGM-corrected blood glucose levels and body weight for a healthy volunteer. A very strong positive correlation was found between the 25th percentile of blood glucose level and the body weight (correlation coefficient r=0.782). A very strong positive correlation was also confirmed in other healthy volunteers.

As shown in the above results, an increase or decrease in body weight can be inferred from variations (changes) in values such as blood GA level and blood glucose level. GA level, blood glucose level, and related values may be monitored for subjects in need of weight control, and used to motivate them to maintain or improve their lifestyle habits.

In some embodiments, the acquisition of GA levels (invasive or non-invasive glycated albumin measurement) may be performed continuously or discontinuously multiple times over a period of time, and the continuous blood glucose measurement may be performed less frequently or for a smaller number of times than the acquisition of GA level. For example, the GA level may be measured every 2 weeks for 10 years, while the AGP may be acquired from CGM measurements for 2 weeks every year, and the variation data or the statistical value of the glucose levels for 10 years may be obtained from the GA levels. In some embodiments, the GA level may be acquired multiple times over a period of time, and a relationship table or correlation may be created which indicates the relationship between these GA levels and statistical values, such as the mean glucose level or the variation range, at the AGP corresponding to or associated with each GA level. In some embodiments, the continuous blood glucose measurement may be repeated to generate a relationship table or correlation which indicates the relationship between a statistical value, such as the mean glucose level or the variation range of the AGP, and the GA level acquired corresponding to or in association with the continuous blood glucose measurement period. In some embodiments, based on the correlation between the GA level and the statistical value of the glucose level by AGP (for example, the mean glucose level), a conversion equation may be made, to acquire the GA level for a period other than the period during which the continuous blood glucose measurement was actually performed, or for a period during which the continuous blood glucose measurement was not actually performed, and obtain the mean glucose level from the acquired GA level.

In some embodiments, by referring to GA levels (first data) or statistical values (for example, range of variation) of continuous blood glucose measurements (CGM) or the like, and all or part of the second data related to subject's previous first data, lifestyle habit, or the like, using machine learning or deep learning, associations between them may be searched for or found. For example, with respect to values or behaviors or changes in variation range of GA level or CGM, the presence or absence of an association with specific factors or items of personal data, health-related data, lifestyle-related data (in this specification, simply referred to as lifestyle-related data or the like), whether the relationship is strong or weak, or the like may be found.

In some embodiments, the system or electronic devices may store variation data or statistics of glucose levels, which correspond to or converted from GA levels. In some embodiments, a relationship of a change in variation data or a statistical value such as a variation range of glucose level obtained based on the GA level or obtained by estimation or a statistical value thereof, with other second data may be obtained or generated. In some embodiments, an association of variation data or a statistical value such as glucose levels obtained by estimation from measured GA levels and changes in those values with other second data may be stored as third data.

The search for the association between the variation range of GA level and CGM and other life habit data or the like may be performed on the same subject, may be performed between different subjects, and may be performed on a plurality or a large number of subjects. These searches and the like may use artificial intelligence. For example, even for the same the constitution, eating habit of individuals, or the group they belong to, the variation range of glucose level due to CGM may be large for some people and small for other people, even though they have the same GA level or mean glucose level. It is expected to find associations or laws that a subject with a large variation range has a high risk of fast vessel hardening, developing complications, or the like. Large-scale genome analyses have identified genes for each race related to diabetes mellitus. For example, there is a mutation in Japanese-specific GLP-1. By genomic tests, target values such as GA value, HbA1c value, BMI, and blood pressure, and the like may be set, or used as parameters for calculating various risks of complications and an average life.

In some embodiments, associations already found by machine learning or the like may be used to determine risk factors or risk degrees, such as health points, risk of complications, and the like, from statistics such as newly acquired GA levels, glucose levels of continuous blood glucose measurements (CGM) and variation ranges thereof. In some embodiments, the % risk of developing complications or other diseases or health forecasts may be generated by referring to GA levels and the variation range of continuous blood glucose measurements. In some embodiments, further second data may be referred to. The continuous blood glucose measurement may be performed, for example, once or more times a year. The continuous blood glucose measurement may be performed seasonally or in conjunction with life events such as New Year, Christmas, consecutive holidays, and the like. Acquisitions of GA level or invasive or non-invasive measurements of glycated albumin concentration may be performed at a higher frequency than continuous blood glucose measurements and the like.

In some embodiments, a table of relationships may be created, between the variation data or statistical values (for example variation range) of glucose levels estimated based on the measured GA levels and changes in these values over the entire period of measured GA levels acquired multiple times in time series, and parameters related to health forecasts, such risk % of each complication, including medical signs a disease such as arteriosclerosis, and estimated time until a complication is contracted, and the associations of them may be generated. In some embodiments, the lifetime loss amount from complication developed to the average life expectancy may be calculated.

In some embodiments, newly acquired GA levels, glucose levels in CGM, and updated information such as lifestyle habits, and the like may be added to the existing first data and second data, and the analysis may be continued by artificial intelligence, thereby increasing or updating the accuracy of the association information (third data).

In an example, a continuous blood glucose measurement (CGM) may be performed to present the user (for example, prediabetes, healthy person) a diabetes risk % or an estimated time to become diabetes based on the AGP or by referring to data of the AGP (for example, the mean or change in mean glucose level and the variation range or the change in variation range) and the first data. In some embodiments, actions by prediabetes, and healthy persons as subjects to improve their own lifestyle habits may perform a continuous blood glucose measurement (CGM), and may, based on the AGP or by referring to the AGP's data (for example, mean value of the glucose level or the change in average value, and variation rage or the change in variation range) and the first data, generate health forecasts including lifestyle habit, treatment, disease risk % or the estimated period until a certain disease is contracted, or health points on the basis thereof, as output information. Such output information may be provided to users, such as diabetics, prediabetics, or healthy persons. In some embodiments, the disease or disorder with respect to disease risk % may be diabetes that a person who is not a diabetic patient contracts. In some embodiments, the time until diabetes is contracted may be estimated. In some embodiments, the disease or disorder with respect to disease risk % may be a complication of a diabetic patient, a disease that occurs at the same time as diabetes, or the like. The term "complication" as used in the present disclosure includes a fact that a diagnosis result of a complication is actually obtained in medical practice, and broadly includes a new or recurrent disease in addition to diabetes regardless of the presence or absence of the diagnosis result. The risk of contracting or developing such complications is referred to as the risk % of complications. In some embodiments, the time until a complication is contracted may be estimated. As a result, for example, the subject or the user can actually feel the influence on the diabetes risk % or the health point. It is possible to realize the risk of diabetes mellitus, the estimated period until diabetes mellitus is contracted, and the health point rise or fall as a result of the self-action such as the improvement of the lifestyle habits. Diabetic patients can also feel the impact of their behavior on the development of diabetes and maintain or enhance the motivation to avoid or reduce, or even ameliorate, worsening conditions. The present disclosure may illustratively help to maintain or enhance awareness of health and disease situations, and motivation for subsequent actions to be taken and actions to be taken on a continuing or non-continuing basis.

In some embodiments, based on continuous blood glucose measurement or measurement by SAP (Sensor Augmented Pump), reactivity and physiological action on a drug of a subject may be determined, and a prescription of a drug or a drug administration proposal may be made. In some embodiments, a GA measurement may be performed substantially simultaneously with a continuous blood glucose measurement. In some embodiments, a GA measurement may be performed substantially continuously or non-continuously from a continuous blood glucose measurement. In some embodiments, on the basis of the GA level at the time of a continuous blood glucose measurement (initial GA level), the GA level thereafter, or a change or variation characteristic thereof (also simply referred to as "change in GA level"), proposals for lifestyle habit or treatment may be made.

In some embodiments, medication information in the second data may be referred to, to suggest a subsequent medication or prescription depending on the GA level, the trend of the GA level, the value associated therewith, or a combination thereof, etc.

In some embodiments, medication information for type 2 diabetes in the second data may be referred to. In some aspects, a medical history within the second data may be referred to.

In some aspects, if the medication information of second data of a type 2 diabetic patient includes a history of receiving sulfonylurea, a type of oral hypoglycemic agent, candidates may be proposed in the order of α-GI, a DPP-4 inhibitor (alert to hypoglycemia), and an SGLT2 inhibitor, when the patient's GA level is determined to be above the limit and postprandial blood glucose level is high.

In the present disclosure, proposing in order may include, in one example, proposing candidate drugs in a safe order in which hypoglycemia is unlikely to occur or in an order in which other side effects are unlikely to occur.

In some examples, the limits of GA level may be determined from conditions such as age, sex, history of diabetes mellitus, complication, genomic information, HbA1c levels, converted HbA1c levels, CGM-data, fasting glucose levels, postprandial glucose levels, insulin-resistance (HOMA-R), insulin-secreting capacity, body condition and the like.

In some aspects, if the medication information of second data of a type 2 diabetic patient includes a history of receiving sulfonylurea, a type of oral hypoglycemic agent, prescription drugs to be safely and inexpensively add may be proposed in the order of a biguanide drug and a DPP-4 inhibitor (alert to hypoglycemia) when the patient's GA level is above the limit and the fasting glucose level is high.

In some aspects, if the medication information of second data of a type 2 diabetic patient includes a history of administration of a biguanide drug, additional drugs may be proposed, in the order of α-GI, a glinide drug, a DPP-4 inhibitor (alert to hypoglycemia) and an SGLT2 inhibitor when the GA limit value determined by referring to the second data is exceeded and the postprandial blood glucose level is high, and in the order of a thiazoline drug, a DPP-4 inhibitor (alert to hypoglycemia) and an SGLT2 inhibitor when the fasting blood glucose level is high.

In some aspects, if the medication information of second data of a type 2 diabetic patient includes a history of administration of a thiazoline drug, it may be proposed to administer in the order of α-GI and a glinide drug when the GA limit value determined by referring to the second data is exceeded and the postprandial blood glucose level is high.

In some aspects, if the medication information of second data of a type 2 diabetic patient includes a history of administration of a thiazoline drug, it may be proposed to administer a biguanide drug as an additional drug when the GA limit value determined by referring to the second data is exceeded and the fasting blood glucose level is high.

In some aspects, if the medication information of second data of a type 2 diabetic patient includes a history of administration of a DPP-4 inhibitor, it may be proposed to administer in the order of α-GI and a glinide drug, when the GA limit value determined by referring to the second data is exceeded and the postprandial blood glucose level is high.

In some aspects, if the medication information of second data of a type 2 diabetic patient includes a history of administration of a DPP-4 inhibitor, it may be proposed to administer additional drugs in the order of a biguanide drug, a thiazoline drug and an SGLT2 inhibitor when the GA limit value determined by referring to the second data is exceeded and the fasting blood glucose level is high.

In some aspects, if the medication information of second data of a type 2 diabetic patient includes a history of administration of an SGLT2 inhibitor, it may be proposed to administer α-GI when the GA limit value determined by referring to the second data is exceeded and the postprandial blood glucose level is high.

In some aspects, if the medication information of second data of a type 2 diabetic patient includes a history of administration of an SGLT2 inhibitor, it may be proposed to administer additional drugs in the order of a biguanide drug and a DPP-4 inhibitor (alert to hypoglycemia) when the GA limit value determined by referring to the second data is exceeded and the fasting blood glucose level is high.

In some aspects, if the medication information of second data of a type 2 diabetic patient includes a history of administration of a GLP-1 receptor agonist, it may be proposed to administer α-GI when the GA limit value determined by referring to the second data is exceeded and the postprandial blood glucose level is high.

In some aspects, if the medication information of second data of a type 2 diabetic patient includes a history of administration of a GLP-1 receptor agonist, it may be proposed to administer additional drugs in the order of a biguanide drug and an SGLT2 inhibitor when the GA limit value determined by referring to the second data is exceeded and the fasting blood glucose level is high.

In some embodiments, reference may be made to medication information in the case of a type 1 diabetes or type 2 diabetes in the second data.

In some aspects, in the case of a type 1 or type 2 diabetic patient, it may be proposed to administer α-GI or a glinide drug, to patients who are receiving an injections a day of persistent lytic insulin (glargine or detemir) at dinner or before going to bed in addition to oral hypoglycemic agents, when the GA level exceeds the GA limit value determined by referring to the second data, and the postprandial blood glucose level is high.

In some aspects, in a case of type 1 or type 2 diabetic patients, it may be proposed to administer additional drugs in the order of a biguanide drug and a DPP-4 inhibitor to patients who are receiving an injections a day of persistent lytic insulin (glargine or detemir) at dinner or before going to bed in addition to oral hypoglycemic agents, when the GA level exceeds the GA limit value determined by referring to the second data, and the fasting blood glucose level is high. As an example, in this case, an alert to nighttime hypoglycemia may also be provided.

In some aspects, in a case of type 1 or type 2 diabetic patients, it may be proposed to administer α-GI or a glinide drug, to patients who are receiving one injections per day of persistent lytic insulin (glargine or detemir) in the morning in addition to oral hypoglycemic agents, when the GA level exceeds the GA limit value determined by referring to the second data, and the postprandial blood glucose level is high.

In some aspects, in a case of type 1 or type 2 diabetic patients, it may be proposed to administer in the order of a biguanide drug and a DPP-4 inhibitor, to patients who are receiving an injections a day of persistent lytic insulin (glargine or detemir) in the morning in addition to oral hypoglycemic agents, when the GA level exceeds the GA limit value determined by referring to the second data and the fasting blood glucose level is high.

In some aspects, in a case of type 1 or type 2 diabetic patients, it may be proposed to administer a DPP-4 inhibitor to patients who are receiving an injections a day of persistent lytic insulin (glargine or detemir) in the morning in addition to oral hypoglycemic agents, when the GA level exceeds the GA limit value determined by referring to the second data and the fasting/postprandial blood glucose level is high. As an example, if the patient is concomitantly taking an SU drugs, an alert to hypoglycemia may also be provided.

It has been clarified that genomic information of about 80 gene mutations involved in diabetes mellitus differs depending on race and also differs among individuals. It is believed in a theory that such differences in genomic information contribute to individual differences, especially in the effects of various diabetic drugs with different mechanisms of action.

On the other hand, it is thought in a theory that factors such as various indices indicating the condition of diabetes mellitus (insulin secretory ability, insulin resistance, HbA1c level, and the like), observance status of exercise therapy and diet therapy, physical condition such as sick day and the like are intertwined with each other and affect the variation range of blood glucose level. Daily blood glucose levels and their variations can be measured by CGM to monitor the variations. As a result, the inventors have determined that the GA level is correlated with various indices (AGP, area under the blood glucose curve, postprandial hyperglycemia frequency, and the like) obtained from CGM. The limit value of the above GA level is in the range of 15.6-25.0%, and values such as 25.0%, 22.5%, 18.3%, 16.5%, and 15.6% are selected depending on the status of glycemic control obtained from CGM.

As noted above, by proposing or alerting physicians and health care professionals to administer hypoglycemic agents, it can, or can help to, bring the GA levels closer to the normal range of healthy individuals (<15.6%).

In some embodiments, if the GA level at the time of continuous blood glucose measurement or the change or variation characteristic thereof is within a predetermined range or an appropriate range, it may be generated as output information or notified to the user that it is good, or the next continuous blood glucose measurement (CGM) may not yet be performed, or it may not be notified at all about the need for the next continuous blood glucose measurement. In some embodiments, if the GA level after the continuous blood glucose measurement or the change or variation characteristic thereof is not within a predetermined range or an appropriate range, output information indicating that a continuous blood glucose measurement should be performed again in the subject may be generated or notified to the user. In some embodiments, when a change such as a rapid decrease in GA level is observed after a continuous blood glucose measurement, the user may be notified that the mean blood glucose level may be rapidly changing, that the blood glucose level at the time of hypoglycemia may be extremely low, or the like, and the next continuous blood glucose measurement may be performed to acquire an AGP, and the user may be notified that the blood glucose change should be checked again. In some embodiments, in the event of a sudden change, such as a decrease in GA level, the user may be notified of an alert or suggestion related to health, lifestyle habit, or the like, such as the likelihood that weight loss or changes will occur, a decrease in muscle mass associated with weight loss, or an alert to locomotive syndrome, injuries, or illnesses.

Continuous/continuous blood glucose measurement or AGP thereby is an invasive method, which is psychologically and economically burdensome for the subject, and it is difficult to continue for a long period of time. On the other hand, if a statistical value (mean value, standard deviation, or the like) of the glucose level can be obtained from a non-invasive GA measurement, the burden on the subject can be reduced.

In some embodiments, if the second data related to the subject includes information on gestation, artificial dialysis, anaemia, erythropoietin-treated, anaemia-treated, renal dysfunction, elevated urea, iron deficiency, vitamin B12 deficiency, folate deficiency, haemoglobinopathies, blood transfusions, metabolic acidosis, haemolysis of the sample at the time of actual HbA1c determination and the like, the HbA1c level may be an outlier, therefore the output information such as misdiagnosis checking, (adjudication, provision of alert information, etc.) may be marked with alert.

In some embodiments, output information may be information that may affect the behavior or behavioral modification of the subject or user. GA levels have an average half-life of 17 days and are an indicator particularly susceptible to human motivation maintenance and behavioral alterations.

In some embodiments, the first data and the second data may be used to diagnose a disease related to blood glucose level. In some embodiments, the first data and the second data may be used to generate advisory information regarding a disease or health condition related to blood glucose level. In some embodiments, diagnostic algorithms performed by or to be performed by a physician may be used to diagnose a disease or generate advisory information regarding a disease or health condition. In some embodiments, diagnostic results of a disease or advice information regarding a disease or health condition may be generated as output information or further provided to the user.

In some embodiments, output information may include graphs of GA levels and changes in GA levels, GA-converted HbA1c levels, differences from the previous values, mean blood glucose levels, dietary information, health forecasts, plans of measures, health points and the like. In some embodiments, these may be generated by a computer system using AI (artificial intelligence) to analyze the first data and the second data , or even to analyze the third data.

Dietary information may include nutritional intake balance, calories intake, sugar intake mass, and glycemic index (GI value). In some embodiments, a meal menu or a photograph of a meal taken may be transmitted to a computer system as the second data of the subject. In some embodiments, based on these inputs, a computer system may analyze, to generate nutritional balance, calories consumed, sugar mass, and GI values of the meal.

A health forecast may include risk % of becoming diabetic, risk % of diabetes complications (risk % of cardiovascular events, risk % of nephropathy, risk % of neuropathy, risk % of blindness), risk % of becoming cancer, risk % of becoming cognitive impairment, and estimated time to becoming these diseases, life loss amount and the like. In some embodiments, these may be generated by a computer system using AI to analyze the first data and the second data collected from subjects, or even to analyze the third data.

In some embodiments, a measure proposal may be generated by a computer system using AI to analyze past changes in GA levels, meal information, season and the like. In some embodiments, a measure proposal may be used to improve or maintain the subject's health and motivation for continuing GA measurement. In some embodiments, in a state where the GA levels is in the upward trend, measures may be provided to prevent postprandial hyperglycemia, leading to an improvement in GA levels in accordance with the lifestyle habit of the subject.

Health points may be generated as output information to improve or maintain the health of the subject. In some embodiments, a health point may be a point given to a subject or a person associated with the subject, such as a caregiver or family, in response to manipulation of the application, measurement of GA levels, input of health checkup data, input of meal photographs, actions associated with illness or health or the like. For example, the minimum value may be set to 1 point when the application is started and viewed, and the maximum value may be set to 50 points when the GA level is measured. In addition, when the GA level decreases by 0.1, 10 points may be given, and the number of points may be given or added to the magnitude of the act that decreases the GA level, such as 2 points when the above-mentioned measures are adhered to, for example, when 8000 steps of walking have been confirmed, 4 points at 12000 steps, 1 point at 80 kcal reduction of meal units, etc.

A "user" receiving a provision of output information may be a subject, may not be a subject, and may include a subject and a non-subject. A "user" may be a person who actually uses a sensor, may be a person who is making measurements of the sensor of himself or of others, may be a person who intends to make measurements and may be obliged or recommended to make measurements or the like. A "user" may be a physician, a medical institution, a pharmacist, a pharmacy, a local government, a government, a work company, a service provider, an insurance provider, a family member, a supplier of other body fluids (subject), or an individual or corporation, an enterprise, an organization or the like that requires, shares, or purchases other health data, may be one or more entities, may be multiple entities or may be multiple entities.

Output information may be generated in the form of information that can be recognized by the user's five senses, a sensor or a computer. Output information may be, for example, visual information such as numerical values, sentences, graphs and images, auditory information such as sound, music, voice and voice messages, tactile information such as Braille or Braille display, odor sense information, taste sense information and a mixture thereof and the like. Output information may be electronic, optical, electromagnetic or a mixture thereof.

In some embodiments, as the visual output information, items selected by the user among the GA levels, the difference from the previous values, the converted HbA1c levels, the converted mean blood glucose levels (HbA1c conversion: 36 days average), and the estimated mean blood glucose levels (GA conversion: 17 days average) may be displayed as numerical values and characters.

For example, various display items (icons) may be prepared on a display such as a smartphone. Items corresponding to the output information related to the first data and the second data collected by the user can be set to be displayable, and the user can arbitrarily select and display desired items in the range. For example, an application may be used to provide output information.

In some embodiments, the output information may be provided to the user based on an indication of the user's intention. In some embodiments, the output information may be configured to allow a user to access, acquire, or view via a terminal, an electronic device or a server. In some embodiments, the output information may not be indicated by the user each time when it is provided. In some embodiments, the output information may be provided based on time, conditions, or the like that the user has preset or agreed upon. In some embodiments, the output information may be provided independent of the intent of the user or based on the decision of the provider. In some embodiments, the provider may be determined by a human or by using a computer in determining the provision of output information. In some embodiments, the intent of the user and the decision of the provider may be used collectively to determine the provision of output information.

In some embodiments, an alert or the like that glycated albumin should be measured may be notified to the user.

The notification may be performed at a predetermined or appropriate timing. For the measurements subsequent to the first time, the notification may be performed at regular or irregular intervals. The interval between any two measurements may be 3 days, 4 days, 5 days, 6 days, 7 days, 10 days, 15 days, 20 days, 30 days, 40 days, 50 days, 60 days, 1 week, 2 weeks, 3 weeks, 4 weeks, 5 weeks, 6 weeks, 7 weeks, 8 weeks, 9 weeks, 10 weeks, 1 month, 2 months, 3 months, 4 months, 5 months, 6 months, or any other period. For measurement of glycated albumin, it may be notified at a period such as 7 days, 10 days, 15 days, 20 days, 1 weeks, 2 weeks, 3 weeks, and the like.

In some embodiments, it may be notified that the timing to collect the body fluid is approaching, that it is the timing, that the timing is past, or such. It is also possible to notify the subject, the collector or the user that the timing at which the body fluid is to be collected is approaching, for example, 1 day before, 2 days before the time point at which the body fluid is to be measured, or the date and time set in advance by the subject, the collector or the user. If the sampling and the measurement have not been performed even though the timing at which the body fluid should be collected has passed, it may be notified to the effect.

In some embodiments, a computer may be used to search for, discover, or calculate associations and correlations, and generate them as third data or information.

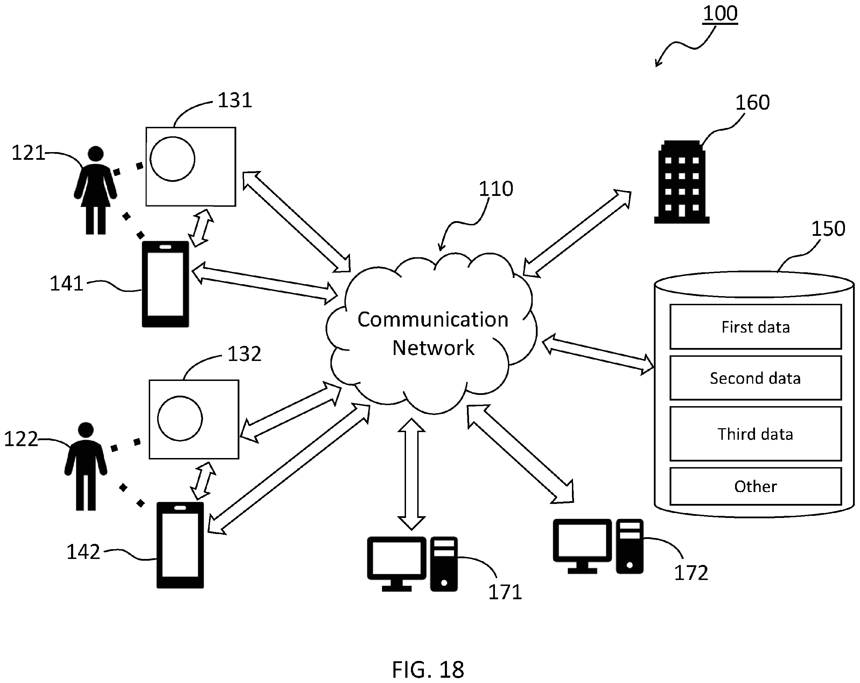

FIG. 18 shows a block diagram of a medical information or healthcare management system 100 associated with GA levels, according to some embodiments. In the system 100 shown in FIG. 18, each device is connected by a communication network 110. A computer for transmitting and calculating information may be performed by a single CPU, but in FIG. 18, a plurality of CPUs are arranged in each device, which cooperate to collect, manage, store, calculate the data, and the like.

The subjects 121,122 measure their own body fluids using GA measuring devices 131,132, respectively. The subjects 121,122 in FIG. 18 use the smart devices 141, 142 as display devices or communication devices. The smart devices 141,142 are connected to the GA measuring devices 131,132, directly, that is, wirelessly or wiredly without passing through a communication network. Therefore, the subjects 121,122 can directly know the GA levels and the transition tendency of the GA levels as the measurement results through the smart devices 141,142.

The GA levels obtained by the measurements of the subjects 121,122 and the time information of the measurement times are transmitted as first data from the GA measuring devices 131,132 or the smart devices 141,142 to memory 150 through the communication network 110 and stored. The memory 150 also stores the second data.

Each configuration of the system of FIG. 18 is connected to an AI analysis center 160 via the communication network 110. The AI analysis center 160 has a function of collecting the first data and the second data stored in the memory 150 and performing AI analysis by machine learning. The AI analysis center 160 accesses the memory 150, analyzes the first data and the second data, and generates analysis results as output information. The output information is transmitted to the smart devices 141,142 of the subjects 121,122 via the communication network 110. Thus, the output information is provided to the subjects 121,122.

The artificial intelligence of the AI analysis center 160 can conduct calculations on a vast amount of the first data and the second data by using artificial intelligence, machine learning or deep learning. The AI analysis center 160 can legally access and refer to the second data stored in memory (not shown) other than the management system network.

The AI analysis center 160 can search for and discover associations and correlations between the first data and the second data. The correlations or data found are stored in the memory 150 as third data. The third data is used for output information.

The output information, including the first data, the second data, and the third data, may also be transmitted via the communication network 110 to non-subject users 171,172, such as medical institutions, public institutions, patient organizations, insurance companies, data companies, and the like. Output information and information or data generated in generating output information is stored in the memory 150.

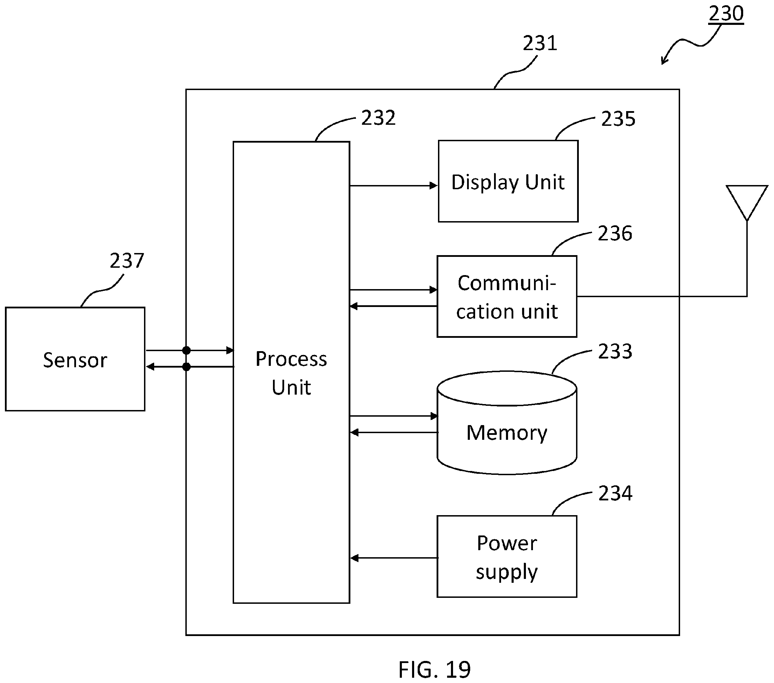

FIG. 19 shows a block diagram of a GA measuring device 230. In the GA measuring device 230 shown in FIG. 19, a process (arithmetic) unit 232 in a main body 231, a memory 233, a power supply 234, a display unit 235, a communication unit 236 are disposed and connected to each other. A sensor 237 is interchangeably or removably mounted to the main body of the GA measuring device. The process unit 232 controls the sensor 237 and receives an output signal from the sensor 237. The process unit 232 performs operations on signals from the sensor 237 and sends the calculation results to the display unit 235, the communication unit 236, and the memory 233. The display unit 235 displays the received signals to the user of the GA measuring device 230. The communication unit 236 transmits the signals received from the process unit 232 to the outside wirelessly (FIG. 19) or wiredly (not shown). Further, the communication unit 236 transmits signals received from the outside to the process unit 232. The memory 233 stores signals or data in an electrical, optoelectronic, or electromagnetic form. The memory 233 stores the signals received from the process unit 232 and provides data or the like to the process unit 232 in response to a command of the process unit 232. The data in the memory 233 is added, deleted, changed, or the like according to the command of the process unit 232. The power supply 234 may be a rechargeable or interchangeable battery (FIG. 19) and may be a power input element from an external power source (not shown). The power supply 234 provides power to the process unit 232. Further, the power supply 234 supplies power to the display unit 235, the communication unit 236, the sensor 237, and the memory 233 via the process unit 232 (FIG. 19) or directly (not shown).

The sensor 237 acquires time information of collection or measurement of body fluids as well as measurements of glycated albumin concentration or measurements of albumin concentration. It also has the function of managing the sensors used or to be used. The GA measuring device 230 acquires, for example, time information such as year, month, date, hour minute at which a measurement was done or tear was collected, and event information and management data such as measurement values including glycated albumin concentrations and albumin concentrations.

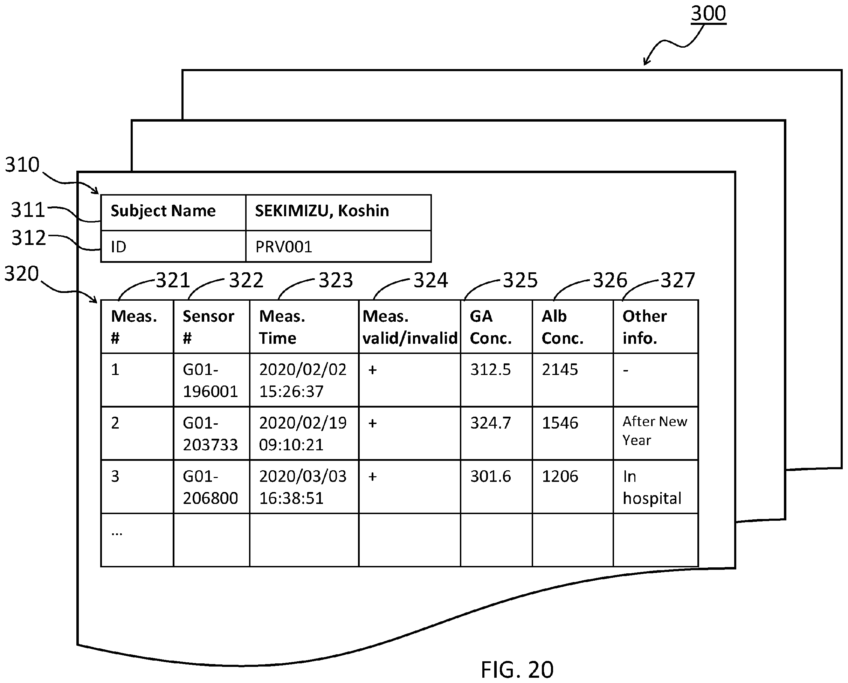

FIG. 20 schematically shows a structure or configuration of the data (first data, primary data) collected by a GA measuring device, or the structure of the first data. The first data is stored as a data array. The data (first data, primary data) collected by a GA measuring device includes information 310 identifying the subject and information 320 relating to the measurement. As the information 310 for identifying the subject, for example, the name of the subject 311 and the ID of the subject 312 are included. As information 320 related to measurements, it contains measurement time information (measurement time) 323, glycated albumin concentration 325, and albumin concentration 326. In addition to this, the first data may include information such as measurement number 321, sensor number (sensor management information) 322, whether the measurement was valid or had an error (measurement valid/invalid information) 324, and other measurement-related information 327. The first data is collected on each subject or in a form associated with the subject.

The GA measuring device 230 shown in FIG. 19 can temporarily record these data in the memory 233 inside the main body.

The time information and the event information may be saved, stored, or recorded as a data string or a data matrix.

The GA measuring device 230 of FIG. 19 can also manage and record management data. Management data may include, without limitation, ID of measuring device main body, manufacturing number of measuring device main body, sensor manufacturing number, sensor lot number, sensor manufacturing date, sensor calibration value, sensor expiration date, remaining number of measurements of the sensor, temperature at the time of measurement, error history, remaining life of the battery, communication code, and the like.

The GA measuring device as shown in FIG. 19 has the arithmetic analysis block (process unit) 232, which can perform the arithmetic analysis. The GA measuring devices 131,132,230 or the smart devices 141,142 may perform relatively simple arithmetic analysis, and the AI analysis center 160 may perform more advanced arithmetic analysis such as arithmetic analysis using artificial intelligence or machine learning on big data. In the arithmetic analysis block 232 in the GA measuring device as shown in FIG. 19, for example, the GA level, the converted HbA1c level, the comparison with the previous value, and the calculation of the mean blood glucose level can be calculated by the following equations.

| | |
|---|---|
| • | GA level (%) = glycated albumin concentration (mg/dL)/albumin concentration |
| | (mg/dL) × 100(%) |

- Estimated blood GA level (%) using tear GA level (%) = a × [tear GA level (%)]α + b
- Difference from previous value = previous GA level - current GA level
- Converted HbA1c level (%) = GA level × 0.245 + 1.73
- Estimated mean blood glucose level (HbA1c conversion: average over the past 36 days) = 28.7 × converted HbA1c-46.7
- Estimated mean blood glucose level (GA conversion: average over the past 17 days) is displayed from the data table of GA levels and mean blood glucose levels.

The above arithmetic expressions are exemplary, and other functions in which the correlation between the respective parameters is defined may be used, or other values may be calculated.

Also, the arithmetic analysis block 232 can calculate the mean blood glucose level for the past two weeks from the GA level. Such arithmetic analysis can also be performed by the smart devices 141,142 or the AI analysis center 160.

The GA measuring device 230 as shown in FIG. 19 includes a means (communication unit) 236 for communicating the above data with the communication network 110, the smart devices 141,142 and the like (see FIG. 18) periodically or optionally as required. The transmitted data may be displayed on an electronic device terminal having a display function such as a personal computer or the smart devices 141,142 (see FIG. 18). The measurement results may be displayed on the display unit 235 of the GA measuring device main body 231.

For example, output information may be generated in response to real-time or time-varying personal thinking, emotions, or desires obtained from a smart device or the like. For example, when a subject wants to eat ramen, the use of a smart device can alert him before eating that eating ramen can have a negative impact on his health condition. Alternatively, information may be provided that ramen should not be eaten, or that ramen should not be eaten completely. Further, the way of providing information may be direct, gentle, or appropriately adjusted according to the user's preference. Further, based on the first data, or the first data and the second data, it is possible to provide future changes in health status, that is, health forecast. Alternatively, it is possible to provide notifications, contacts, information provisions for coaching about lifestyle habits, or improving or maintaining the motivation. Alternatively, it may be prompted to go to hospital.

The present system can collect the second data and analyze it by machine learning from the control state of the blood glucose levels at the time of GA measurements in accordance with the first data, and predict the risk of diabetes mellitus or its complications and other diseases occurring in the future. As the second data, in particular, health data may be referred to.

The operations, actions, calculations, analyses, communication of information and the like included in the present disclosure may be performed by a computer or computer system. A computer or the like may include input means, a processor, a memory means for storing a database, and a display means. The computer itself, the processor, the memory, the storage medium, or the like may be disposed each as one component, may be configured to have a plurality of components, may be installed in one location, may be installed in a plurality of locations separately, and may be configured to be connected via a communication means such as a network, or may be configured to be connected as necessary.

The system may notify the collector of an act to be performed with respect to collection or measurement of body fluid. At the time of notification, actions to be taken in connection with collection or measurement of body fluid may be notified, such as the number of the sensor to be used, how to use the sensor, precautions at the time of measurement, the method of transmitting and storing measurement data after the measurement, or the like.

The subject measures the GA level for his or her own body fluid. The system acquires the measured value from a sensor or a GA measuring device together with its measurement time information. By performing this GA level measurement once or multiple times, first data including glycated albumin concentration and albumin concentration (GA related information) and time related information can be collected. The first data is stored in a memory by a computer system.

The second data may also be provided from the subject, but may also be collected from other than the subject (non-subject). A "non-subject" may be a diabetic patient, a prediabetic, a patient with a glucose complication, a subject, a user, their family or related persons or an agent, a party such as a company, or an organization. The second data may be collected from a non-subject, may be collected from a subject, and may be collected from a subject and a non-subject.

The system may refer to the first data and the second data. The system may refer to second data items associated with the first data items of interest. In some embodiments, the first data or measured GA levels may be evaluated by referring to the second data for false diagnosis checking. In some embodiments, the second data may be referred to during AI analysis. In the analysis, in addition to the first data and second data, the system may refer to the third data indicating the associations of the first data and second data as described later, or other data. The third data may be stored in a memory.

The system may generate output information based on the first data and the second data. In some embodiments, associations or correlations between the first data and the second data may be searched. In some embodiments, this association may be stored as third data in a memory and provided to the user as output information.

In some embodiments, when, or before or after, providing output information to a user, a notification of a measurement of a sample or a provision of new second data may be requested.

In some embodiments, the acquisition of the second data and the provision of the output information may be performed without necessarily being linked to the acquisition of the first data.

The second data may be acquired in response to a notification, a request or the like from the system, or may be voluntarily or automatically collected from a subject, a collector, a user, or another individual or organization. In some embodiments, an event during life, such as a subject's meal or exercise, may be acquired as second data. In some embodiments, the subject may voluntarily enter the second data into a smart device. In some embodiments, when a subject uses a smart device or the like or when a communication event occurs at the time, the system may react to provide the subject, collector, or user with information or suggestions that may be appropriate or useful. In some embodiments, the system may refer to and acquire health care data stored in or connected to a smart device or the like of a subject. In some embodiments, the system may request access to the data or seek consent from a subject or the like. In some embodiments, meal information may be generated by the subject acquiring a meal photograph using a smart device or the like, and calculating nutrition, sugar content, calories or the like from the photograph. In some embodiments, a subject may acquire an exercise condition through a smart device or the like by the system. In some embodiments, the system may acquire exercise related information at a sports club, such as a gym or a swimming pool.

In some embodiments, the system may generate and notify the user of a five-step health forecast, diabetes risk %, cardiovascular event risk %, nephropathy risk %, neuropathy risk %, blindness risk %, lifetime loss amount conversion, or the like, as output information.

The system may also propose ways to effectively reduce these risks to suit the individual characteristics, population characteristics, and individual lifestyle habits of service recipients. For example, a suggestion of exercise associated with a meal may be made, such as a slow walking of 5,000 steps within 30 minutes after a meal. Alternatively, for example, a meal suggestion may be made such that at the next meal, 100 g of vegetables should be eaten first, followed by 80 g of rice slowly in 10 minutes, or the like.

In some embodiments, if there is an effect at the succeeding GA measurement, the displayed risk is improved, and a health point is awarded. The health points may be calculated in accordance with items such as lifestyle habits while considering changes in the GA levels or the like. For example, it is possible to find out the degree of influence of each item of lifestyle habits according to not only the items of lifestyle habits rigidly reflected in the health points, but also the results that monitor the GA levels, and the constitution and habits that suit each individual.

In some embodiments, health points, other medical data, or first, second, third data, or the like may be shared in whole or in part via a computer system with organizations such as insurance companies, local governments, medical institutions, dispensing pharmacies, and the like.

By displaying the results of the analysis of the machine learning to the service recipient, various services can be provided to motivate the recipient to prevent a disease or prevent the development of a disease.

A program or a computer program according to some embodiments of the present disclosure may cause a computer to execute each step included in the measurement methods described in the present application, or may cause a computer to read and execute the step. A computer-readable storage medium according to some embodiments of the present disclosure may store a program or a computer program for causing a computer to execute each step included in the measurement methods described in the present application.

Some embodiments of the present disclosure are data structures comprising data associated with the GA level or an array of data. The data structure may be provided so that a computer or computer system can refer to the first data and the second data, generate output information, and provide it to the user.

In some embodiments, the first data may include glycated albumin concentrations and albumin concentrations in a plurality of body fluids and their measurement times or times of collection of body fluids. In some embodiments, the first data may be configured so that measured values may be added for each measurement of glycated albumin or a GA level.

In some embodiments, the data structure may be configured such that a computer or the like can refer to the first data and the second data, generate output information including at least one of: GA level, converted HbA1c level, comparison with previous values, mean blood glucose level; estimates of these values during one or more measurement periods in the past; predicted values of these values for one or more future measurement periods; trend information of the lifestyle habits in the past; risk of developing diabetes or its complications and other diseases in the future; and recommendations on lifestyle habits, and can provide the output information to the user.

In some embodiments, the data structure may be configured to allow third data to be added for each generation thereof. In some embodiments, the third data may be configured to be accessed by a computer or the like, referred to, and based upon which the output information may be generated and provided to the user.

According to some embodiments of the present disclosure, potentially, data regarding non-invasively obtained GA is used, so that the burden on the subject is reduced as compared with an invasive method. Therefore, it is possible to acquire, analyze a larger amount of data over the medium to long term, and provide useful information to the user for a longer period of time, not just for a single shot.

In some embodiments of the present disclosure, data based on GA levels are likely to contribute to maintaining or improving a subject's health care motivation. It is easier to understand when compared with other indicators (markers) indicating the control state of the blood glucose levels.

For example, in hospitals, glycated hemoglobin (HbA1c) is measured by blood sampling of diabetic patients or as part of a blood test during a health checkup. Glycated hemoglobin (HbA1c) is an indicator of the glycation rate of red blood cell hemoglobin of the subject or the like. The interval between visits for diabetic patients is usually 1 to 3 months. Because the half-life of red blood cells is about 36 days, this marker is an indicator of blood glucose levels over a period of 1 to 2 months. The HbA1c level is measured once every 1 to 3 months for diabetic patients and once a year for healthy people, such as health examinations. Thus, the measurement of HbA1c is invasive, and the measurement intervals are several months, which makes it physiologically and psychologically unsuitable to maintain the motivation of the subject, for example, between visits.

Since the methods, systems, data structures, and the like of the present disclosure are based on GA levels obtained by non-invasive measurements, physiological and psychological burden on the subject or the like is reduced to the utmost, and it is easy to continue medium to long term measurements. Moreover, GA, with a half-life of about 2 weeks, is optimal as a marker for observing or tracking variation over the medium to long term. Therefore, according to the present disclosure, physiological and psychological burden of a subject or the like is extremely reduced, and the motivation for maintaining and improving health of the subject or the like can be maintained and improved.

According to some embodiments of the present disclosure, potentially or exemplarily, it is possible to provide a service that ascertains the status of a disease related to the lifestyle habits of an individual serving as a service recipient, and enhances motivation to improve the control state of blood glucose levels by presenting a disease risk and an optimal solution of a countermeasure based on characteristics of the individual, such as time-varying characteristics of the individual, characteristics of a country or a region, characteristics of a group similar to the individual, a family history of the individual, a group having a background of genetic information or the like.

According to some embodiments of the present disclosure, potentially or exemplarily, the blood glucose control condition can be provided to the user based on the first data of the time series of the GA measurement values in tears or saliva which can be collected in a non-invasive manner. Moreover, the first data is also analyzed using AI or the like by integrating with other collectible health-related information, which makes it possible to provide a service including methods for preventing diabetes, progress of diabetes, methods for improving it, and methods for preventing complications, useful healthcare related information and suggestions, and the like.

The present disclosure also includes the following embodiments:
A01a
   A method of healthcare management related to a blood glucose level, a method of providing healthcare information, a method of managing diabetes risk, or a method of preventing complications in diabetic patients, the method comprising:
   collecting first data including an albumin concentration and a glycated albumin concentration in a body fluid, from at least one human or at least one mammal;
   generating output information associated with the collected first data; and
   providing a user with the generated output information.
A01b
   A method of healthcare management related to a blood glucose levels, a method of providing healthcare information, a method of managing diabetes risk, or a method of preventing complications in diabetic patients, the method comprising:
   collecting first data including an albumin concentration, a glycated albumin concentration obtained by a measurement in a body fluid of a subject, and time information of the measurement, and inputting the first data into a computer system;
   using the computer system to generate output information related to a GA level based on the first data; and
   providing a user with the generated output information from the computer system.
A01c
   A method of healthcare management related to a blood glucose level, a method of providing healthcare information, a method of managing diabetes risk, or a method of preventing complications in diabetic patients, the method comprising:
   collecting first data including a glycated albumin concentration in a body fluid of a subject and inputting the first data into a computer system;
   using the computer system to generate output information related to a GA level based on the first data; and
   providing a user with the generated output information from the computer system.
A01 or A01d
   A method of healthcare management related to a blood glucose level, a method of providing healthcare information, a method of managing diabetes risk, or a method of preventing complications in diabetic patients, the method comprising:
   acquiring first data including a glycated albumin concentration and an albumin concentration in a body fluid of a subject;
   generating output information related to a GA level based on the first data; and providing a user with the generated output information.
A02
   The method of claim A01, wherein said acquiring the first data comprises measuring the glycated albumin concentration in the body fluid of the subject.
A03
   The method of embodiment A01 or A02, wherein said acquiring the first data comprises collecting the body fluid of the subject.
A03b
   The method of any one of embodiments A01a to A03, wherein the body fluid comprises at least one of blood, tears and saliva.
A03c
   The method of embodiment A03, wherein said collecting the body fluid of the subject comprises non-invasively collecting tears or saliva.
A04
   The method of any one of embodiments A01 to A03c, wherein said acquiring the first data comprises acquiring a concentration of albumin in the body fluid of the subject.
A05
   The method of any one of embodiments A01 to A04, wherein said acquiring the first data comprises acquiring time information relating to a secretion, a collection or a measurement of the body fluid of the subject to be measured.
A11
   The method of any one of embodiments A01 to A05, wherein said acquiring the first data comprises measuring the glycated albumin concentration and the albumin concentration in tears or saliva, multiple times.
A12
   The method of any one of embodiments A01 to A11, wherein said acquiring the first data comprises measuring the glycated albumin concentration in tears or saliva multiple times, over a period ranging from two weeks to six months.
A13
   The method of any one of embodiments A01 to A11, wherein said collecting the first data comprises measuring the glycated albumin concentration in tears or saliva multiple times at an average interval of 7 to 28 days.
A21
   The method according to any one of embodiments A01 to A13,
   wherein said acquiring the first data comprises measuring the glycated albumin concentration and the albumin concentration in tears or saliva multiple times at an average interval of 7 to 28 days, and
   wherein said generating the output information comprises generating information indicating at least one of a GA level, a graph of change in GA level, a converted HbA1c level, a comparison with a previous value, a mean blood glucose level, a variation in mean blood glucose level, a health forecast, a proposed response, and health points, related to a part or all of the plurality of measurements.
A22
   The method of any one of embodiments A01 to A21,
   wherein the output information includes at least one of:
   a GA level, a converted HbA1c level, a comparison with a previous value, an estimated mean blood glucose level, related to a part or all of the period during which the measurement was performed;
   a GA level, a converted HbA1c level, a comparison with a previous value, a predicted mean blood glucose level, related to one or a plurality of periods or points of time in the future; trend information of lifestyle habits in the past;
   a risk of developing future diabetes or its complications and other diseases;
   a recommendation related to lifestyle habits; and
   a recommendation related to taking medications.
A31
   The method according to any one of embodiments A01 to A22, further comprising collecting second data.
A39
   The method of embodiment A31, wherein the second data comprises data relating to a non-subject.
A401
   The method of any one of embodiments A01 to A31, said generating the output information related to the glycated albumin amount comprises:
   referring to second data including at least one of personal data, health-related data, lifestyle habit data, and population data; and
   generating output information using a computer, on the basis of the first data and the second data.
A402
   The method of any one of embodiments A01 to A401, wherein the second data comprises: personal ID ("my number"), age, gender, marital status, number of children, address, residence type, owned cars, owned motorcycles, owned bicycles, job, job type, job position, annual salary, genomic information, belief, health insurance number, fingerprint, voice print, face recognition information, iris recognition information, time-changing personal ideas/emotion/desire/request, input to smart devices and usage information thereof;
   race, nationality, region, district area, seasonal variation factors (seasonal temperature, humidity changes, or the like), holiday periods (including year-end, spring holidays, summer holidays, or the like), means of commutation, religion;
   body weight, height, body fat, maximal blood pressure, minimal blood pressure, heart rate, step count, activity, blood oxygenation, body temperature, room temperature, solar radiation, UV radiation, mean blood glucose levels obtained from continuous blood glucose level measurements (Continuous Glucose Monitoring (CGM), Flash Glucose Monitoring (FGM)) devices, standard deviations, AGPs, and health checkup data (including HbA1c), medical history, prescription medications, medication information, data from health institutions, food and other allergies, family history and health-related data;
   dietary habits (carnivores, vegetarians, and the like), tastes, meal menus, meal photographs, snacks, exercise habits, wake-up time, bedtime, sleeping time, sleep depths, sleep points, active or inactive rest times, meal speed, chewing
   frequency/rate/characteristics, food allergies, religious belief-related dietary rules (Ramadan, and the like), exercise status, exercise items, exercise time, exercise frequency, exercise consumption calories, hobbies.
A403
   The method of any one of embodiments A401 or A402, said using the computer to generate the output information comprises using artificial intelligence to perform machine learning or deep learning on data including the first data and the second data.
A404
   The method of any one of embodiments A401 to A403, wherein the second data includes data relating to the subject.
A405
   The method of any one of embodiments A401 to A404, wherein said generating the output information comprises generating as output information an alert that there is a possibility of a false high value if the second data related to the subject includes information on liver cirrhosis, hypothyroidism, or administration of a thyroid hormone synthesis inhibitor.
A406
   The method of any one of embodiments A401 to A405, wherein said generating the output information comprises generating as output information an alert that there is a possibility of a false low value if the second data related to the subject includes information on nephrotic syndrome, protein-leaking gastroenteropathy, hyperthyroidism, or thyroid hormone therapeutics.
A407
   The method of any one of embodiments A401 to A406, wherein said generating the output information comprises
   if the second data includes HbA1c levels measured at a medical institution, or the like, in the second data relating to said subject, comparing with the GA-converted HbA1c levels of the most recent first data,
   when the measured HbA1c level ≥ the GA-converted HbA1c level, generating output information that the mean blood glucose level is improved,
   when the measured HbA1c level < the GA-converted HbA1c level, generating output information that the mean blood glucose level is deteriorated, and
   if the second data related to the subject includes information related to at least one of gestation, artificial dialysis, anaemia, erythropoietin-treated, anaemia-treated, renal dysfunction, elevated urea, iron deficiency, vitamin B12 deficiency, folate deficiency, haemoglobinopathies, blood transfusions, metabolic acidosis, and haemolysis of the sample at the time of actual HbA1c determination, marking the output information with an alert.
A408
   The method of any one of embodiments A31 to A407, wherein the second data includes variation data of glucose levels obtained by a continuous blood glucose measurement.
A409
   The method of any one of claims A31 to A407, wherein the second data comprises AGP obtained by a continuous blood glucose measurement.
A410
   The method of any one of embodiments A31 to A407, further comprising:
   acquiring GA levels multiple times substantially continuously over a period of time, as the first data; and
   performing continuous blood glucose measurements for a number of times less than the number of times of the GA level acquisition, as the second data, to acquire variation data of glucose levels.
A411
   The method of embodiment A410, further comprising determining a correlation between an average value of the variation data of glucose levels acquired by the continuous blood glucose measurement and the GA level.
A412
   The method of embodiment A411, said determining the correlation comprises referring to all or part of the second data related to the subject and using machine learning or deep learning, to optimize the correlation.
A413
   The method of embodiment A411 or A412, further comprising, based on the correlation, estimating, from the GA level, a mean value of glucose levels for a period other than the period during which the continuous blood glucose measurement was actually performed.
A409b
   The method of embodiment A408, further comprising determining a correlation between the variation data of the glucose levels obtained by the continuous blood glucose measurement and the GA level.
A409c
   The method of embodiments A409b, wherein the correlation between the variation data of the glucose levels obtained by the continuous blood glucose measurement and the GA level comprises a correlation between a 75th percentile value of the glucose levels of the continuous blood glucose measurement and the variation data and the GA level.
A409c
   The method of embodiment A409b, wherein the correlation between the variation data of the glucose levels obtained by the continuous blood glucose measurement and the GA level comprises a correlation between a 90th percentile value of the glucose levels of the continuous blood glucose measurement and the variation data and the GA level.
A409d
   The method of embodiment A409c, wherein the correlation between the variation data of the glucose levels obtained by the continuous blood glucose measurement and the GA level comprises a correlation between a median value of the glucose levels of the continuous blood glucose measurement and the variation data and the GA level.
A409e
   The method of embodiment A409b, wherein the correlation between the variation data of the glucose levels obtained by the continuous blood glucose measurement and the GA level comprises a correlation between a frequency of postprandial hyperglycemia and the variation data and the GA level.
A409f
   The method of embodiment A409b, wherein the correlation between the variation data of the glucose levels obtained by the continuous blood glucose measurement and the GA level comprises a correlation between an area under a curve of the glucose levels obtained by the continuous blood glucose measurement and the GA level.
A409g
   The method of embodiment A409b, wherein the correlation between the variation data of the glucose levels obtained by the continuous blood glucose measurement and the GA level comprises a correlation between an area between curves of the glucose levels obtained by the continuous blood glucose measurement and the GA level.
A409h
   The method of embodiment A409g, wherein the area between the curves of the glucose levels obtained by the continuous blood glucose measurement is,
   an area between a 25th percentile curve and a 75th percentile curve,
   an area between a 10th percentile curve and a 75th percentile curve, or an area between the 2th percentile curve and a 75th percentile curve.
A409i
   The method of any one of embodiments A409b to A409g, said determining the correlation between the variation data of the glucose levels obtained by the continuous blood glucose measurement and the GA level comprises correcting the glucose levels obtained by the continuous blood glucose measurement by using a function of a uric acid level.
A409j
   The method of embodiment A409i, wherein said determining the correlation between the variation data of the glucose levels obtained by the continuous blood glucose measurement and the GA level further comprises determining a correlation between a corrected glucose level by a uric acid level and the GA level.
A409k
   The method of any one of embodiments A409b to A409i, the correlation is acquired as the second data,
A409A
   The method of embodiment A402, further comprising determining a correlation between a body weight and the GA level.
A409B
   The method of embodiment A402 or A409B, wherein a variation in body weight is estimated from the GA level or the variation in the GA level. A414
   The method of any one of embodiments A411 to A413, further comprising storing the correlation as third data.
A415
   The method of any one of embodiments A411 to A414, wherein the correlation comprises at least one of a mean value and a variation range of the glucose levels.
A416
   The method of any one of embodiments A411 to A415, wherein based on the change in GA level acquired after the continuous blood glucose measurement, it is determined whether or not a next continuous blood glucose measurement should be performed, and output information including the determination is generated.
A417
   The method of embodiment A416, wherein by considering the GA level acquired substantially simultaneously with the continuous blood glucose measurement, it is determined whether or not a next continuous blood glucose measurement should be performed.
A418
   The method of embodiment A417, wherein when a rate of change of the GA level acquired after the continuous blood glucose measurement is greater than a predetermined value, output information indicating that the next continuous blood glucose measurement should be performed is generated.
A419
   The method of any one of embodiments A416 to A418, further comprising generating a prescription of a drug or generating output information of an administration proposal in accordance with AGP after the continuous blood glucose measurement.
A419b
   The method of any one of embodiments A01a to A419, further comprising: referring to the prescription and/or medication information in the second data, and generating as output information a proposal for the next prescription and/or the administration, in accordance with the GA level or a trend of the GA levels, a value associated therewith or a combination thereof.
A420
   The method of embodiment A409 or A10, wherein a suggestion regarding a lifestyle habit or a treatment is generated as output information, by referring to the first data and the mean value and the variation range of the glucose levels of the continuous blood glucose measurement.
A421
   The method of embodiment A420, further comprising providing output information including a suggestion regarding the lifestyle habit or the treatment to a diabetic patient, a prediabetic or a healthy person.
A422
   The method of embodiment A409 or A10, wherein by referring to the first data and the mean value and the variation range of the glucose levels of the continuous blood glucose measurement, a diabetes mellitus risk % or an estimated time period until diabetes mellitus is contracted is generated as output information.
A423
   The method of embodiment A422, further comprising providing output information including the diabetes mellitus risk % or the estimated time period until diabetes is contracted, to a prediabetic or a healthy person.
A424
   The method of embodiment A409 or A10, wherein by referring to the first data and the mean value and the variation range of the glucose levels of the continuous blood glucose measurement, a diabetic complication risk % or an estimated time period until a complication is contracted is generated as output information.
A425
   The method of embodiment A424, further comprising providing output information including the diabetic complication risk % or the estimated time period until a complication is contracted, to a diabetic patient.
A51
   The method of any one of embodiments A31 to A419, further comprising notifying a collector of a body fluid that it should be collected.
A52
   The method of embodiment A51, wherein said notifying comprises notifying that the body fluid should be collected at a frequency of substantially once every 17 days or 2 weeks.
A53
   The method of embodiment A51 or A52, wherein said notifying comprises notifying that the timing of collection of the body fluid is approaching, that it is the timing, or that the timing is passed.
A54
   The method of any one of embodiments A51 to A53, wherein said notifying comprises notifying the action to be taken related to a collection or a measurement of the body fluid.
A61
   The method of any one of embodiments A31 to A54, further comprising searching for a correlation between the first data and the second data and generating third data comprising the correlation as an attribute.
A62
   The method of embodiment A61, wherein the output information includes the third data.
A71
   Medication information in the second data.
B01
   Software for performing the method of embodiments A01 to A62.
C01
   A storage medium storing a software for performing the method of embodiments A01 to A62.
D01
   A healthcare management system, the system being configured to be connected to a sensor for measuring a GA level in a body fluid, and
   the system comprising:
   a memory configured to store:
   first data including a glycated albumin concentration and an albumin concentration in a plurality of body fluids, measured by the sensor, and a measurement time thereof, and
   second data including at least one of personal data, health-related data, lifestyle habit data, and population data;
   an analysis center configured to analyze the first data and the second data; and
   a computer configured to provide useful healthcare-related information to a user based on the analysis results by the analysis center.
E01
   A data structure for healthcare management, comprising:
   a first data array configured to allow addition of first data including a glycated albumin concentration and an albumin concentration in a body fluid measured by a glycated albumin sensor and their measurement times; and
   a second data array comprising at least one of personal data, health-related data, lifestyle habit data, and population data, and
   the data structure being configured to, by a computer,
      refer to the first data and the second data,
      generate output information including at least one of:
         a GA level, a converted HbA1c level, a comparison with a previous value, a mean blood glucose level;
         estimates of these values during one or more measurement periods in the past;
         predicted values for one or more future measurement periods;
         trend information of the past lifestyle habits;
         a risk of developing future diabetes or its complications and other diseases; and
         a recommendation on lifestyle habits,
   and provide the output information to a user.
E02
   The data structure for healthcare management of embodiment E01, further comprising a third data array including third data including association information between the first data and the second data.

While several embodiments and examples of the present disclosure have been described above, these embodiments and examples illustrate the present disclosure. For example, each of the embodiments described above has been described in detail in order to explain the present disclosure in an easy-to-understand manner, and dimensions, configurations, materials, and circuits may be additionally changed as necessary. Embodiments in which one or more features of the present disclosure described above are arbitrarily combined are also included in the scope of the present disclosure. It is intended that the appended claims cover numerous modifications to the embodiments without departing from the spirit and scope of the present disclosure. Accordingly, the embodiments and examples disclosed herein have been shown by way of examples and should not be considered as limiting the scope of the present disclosure.

The following items are furthermore disclosed:
1. A method of healthcare management, comprising:
   acquiring first data including a glycated albumin concentration and an albumin concentration in a body fluid of a subject;
   generating output information related to a GA level based on the first data; and
   providing a user with the generated output information.
2. The method of item 1, wherein said acquiring the first data comprises measuring the glycated albumin concentration in the body fluid of the subject.
3. The method of item 1 or 2, wherein said acquiring the first data comprises collecting the body fluid of the subject.
4. The method of any one of items 1 to 3, wherein said acquiring the first data comprises acquiring a concentration of albumin in the body fluid of the subject.
5. The method of any one of items 1 to 4, wherein said acquiring the first data comprises acquiring time information relating to a secretion, a collection or a measurement of the body fluid of the subject to be measured.
6. The method of any one of items 1 to 5, wherein said acquiring the first data comprises measuring the glycated albumin concentration and the albumin concentration in the body fluid, multiple times.
7. The method of any one of items 1 to 6, wherein said acquiring the first data comprises measuring the glycated albumin concentration in tears or saliva multiple times, over a period ranging from two weeks to six months.
8. The method of any one of items 1 to 6, wherein said collecting the first data comprises measuring the glycated albumin concentration in tears or saliva multiple times at an average interval of 7 to 28 days.
9. The method according to any one of items 1 to 8,
   wherein said acquiring the first data comprises measuring the glycated albumin concentration and the albumin concentration in tears or saliva multiple times at an average interval of 7 to 28 days, and
   wherein said generating the output information comprises generating information indicating at least one of a GA level, a graph of change in GA level, a converted HbA1c level, a comparison with a previous value, a mean blood glucose level, a variation in mean blood glucose level, a health forecast, a proposed response, and health points, related to a part or all of the plurality of measurements.
10. The method of any one of items 1 to 9, wherein the output information includes at least one of:
   a GA level, a converted HbA1c level, a comparison with a previous value, an estimated mean blood glucose level, related to a part or all of the period during which the measurement was performed;
   a GA level, a converted HbA1c level, a comparison with a previous value, a predicted mean blood glucose level, related to one or a plurality of periods or points of time in the future; trend information of lifestyle habits in the past;
   a risk of developing future diabetes or its complications and other diseases;
   a recommendation related to lifestyle habits; and
   a recommendation related to taking medications.
11. The method according to any one of items 1 to 10, further comprising collecting second data.
12. The method of item 11, wherein the second data comprises data relating to a non-subject.
13. The method of any one of items 1 to 11, said generating the output information related to the glycated albumin amount comprises:
   referring to second data including at least one of personal data, health-related data, lifestyle habit data, and population data; and
   generating output information using a computer, on the basis of the first data and the second data.
14. The method of any one of items 1 to 13, wherein the second data comprises:
   personal ID ("my number"), age, gender, marital status, number of children, address, residence type, owned cars, owned motorcycles, owned bicycles, job, job type, job position, annual salary, genomic information, belief, health insurance number, fingerprint, voice print, face recognition information, iris recognition information, time-changing personal ideas/emotion/desire/request, input to smart devices and usage information thereof;
   race, nationality, region, district area, seasonal variation factors (seasonal temperature, humidity changes, or the like), holiday periods (including year-end, spring holidays, summer holidays, or the like), means of commutation, religion;
   body weight, height, body fat, maximal blood pressure, minimal blood pressure, heart rate, step count, activity, blood oxygenation, body temperature, room temperature, solar radiation, UV radiation, mean blood glucose levels obtained from continuous blood glucose level measurements (CGM, FGM) devices, standard deviations, AGP, and health checkup data (including HbA1c), medical history, prescription medications, medication information, data from health institutions, food and other allergies, family history and health-related data;
   dietary habits (carnivores, vegetarians, and the like), tastes, meal menus, meal photographs, snacks, exercise habits, wake-up time, bedtime, sleeping time, sleep depths, sleep points, active or inactive rest times, meal speed, chewing
   frequency/rate/characteristics, food allergies, religious belief-related dietary rules (Ramadan, and the like), exercise status, exercise items, exercise time, exercise frequency, exercise consumption calories, hobbies.
15. The method of any one of items 13 or 14, said using the computer to generate the output information comprises using artificial intelligence to perform machine learning or deep learning on data including the first data and the second data.
16. The method of any one of items 13 to 15, wherein the second data includes data relating to the subject.
17. The method of any one of items 13 to 16, wherein said generating the output information comprises generating as output information an alert that there is a possibility of a false high value if the second data related to the subject includes information on liver cirrhosis, hypothyroidism, or administration of a thyroid hormone synthesis inhibitor.
18. The method of any one of items 13 to 17, wherein said generating the output information comprises generating as output information an alert that there is a possibility of a false low value if the second data related to the subject includes information on nephrotic syndrome, protein-leaking gastroenteropathy, hyperthyroidism, or thyroid hormone therapeutics.
19. The method of any one of items 13 to 18, wherein said generating the output information comprises
   if the second data includes HbA1c levels measured at a medical institution, or the like, in the second data relating to said subject, comparing with the GA-converted HbA1c levels of the most recent first data,
   when the measured HbA1c level ≥ the GA-converted HbA1c level, generating output information that the mean blood glucose level is improved,
   when the measured HbA1c level < the GA-converted HbA1c level, generating output information that the mean blood glucose level is deteriorated, and
   if the second data related to the subject includes information related to at least one of gestation, artificial dialysis, anaemia, erythropoietin-treated, anaemia-treated, renal dysfunction, elevated urea, iron deficiency, vitamin B12 deficiency, folate deficiency, haemoglobinopathies, blood transfusions, metabolic acidosis, and haemolysis of the sample at the time of actual HbA1c determination, marking the output information with an alert.
20. The method of any one of items 11 to 19, wherein the second data includes variation data of glucose levels obtained by a continuous blood glucose measurement.
21. The method of any one of items 11 to A41907, wherein the second data comprises AGP obtained by a continuous blood glucose measurement.
22. The method of any one of items 11 to 19, further comprising:
   acquiring GA levels multiple times substantially continuously over a period of time, as the first data; and
   performing continuous blood glucose measurements for a number of times less than the number of times of the GA level acquisition, as the second data, to acquire variation data of glucose levels.
23. The method of any one of items 20 to 22, further comprising determining a correlation between an average value of the variation data of glucose levels acquired by the continuous blood glucose measurement and the GA level.
24. The method of item 23, said determining the correlation comprises referring to all or part of the second data related to the subject and using machine learning or deep learning, to optimize the correlation.
25. The method of item 23 or 24, further comprising, based on the correlation, estimating, from the GA level, a mean value of glucose levels for a period other than the period during which the continuous blood glucose measurement was actually performed.
26. The method of any one of items 23 to 25, wherein the correlation between the variation data of the glucose levels obtained by the continuous blood glucose measurement and the GA level comprises at least one of:
   a correlation between a 75th percentile value of the glucose levels of the continuous blood glucose measurement and the variation data and the GA level;
   a correlation between a 90th percentile value of the glucose levels of the continuous blood glucose measurement and the variation data and the GA level;
   a correlation between a median value of the glucose levels of the continuous blood glucose measurement and the variation data and the GA level;
   a correlation between a frequency of postprandial hyperglycemia and the variation data and the GA level;
   a correlation between an area under a curve of the glucose levels obtained by the continuous blood glucose measurement and the GA level; and
   a correlation between an area between curves of the glucose levels obtained by the continuous blood glucose measurement and the GA level.
27. The method of embodiment A409g, wherein the area between the curves of the glucose levels obtained by the continuous blood glucose measurement is,
   an area between a 25th percentile curve and a 75th percentile curve,
   an area between a 10th percentile curve and a 75th percentile curve, or
   an area between a 2th percentile curve and a 75th percentile curve.
28. The method of any one of items 25 to 27, said determining the correlation between the variation data of the glucose levels obtained by the continuous blood glucose measurement and the GA level comprises
   correcting the glucose levels obtained by the continuous blood glucose measurement by using a function of a uric acid level; and
   determining a correlation between a corrected glucose level by a uric acid level and the GA level.
29. The method of item 14, further comprising determining a correlation between a body weight and the GA level.
30. The method of item 14 or 29, wherein a variation in body weight is estimated from the GA level or the variation in the GA level.
31. The method of any one of items 23 to 30, further comprising storing the correlation as third data.
32. The method of any one of items 23 to 31, wherein the correlation comprises at least one of a mean value and a variation range of the glucose levels.
33. The method of any one of items 23 to 32, wherein based on the change in GA level acquired after the continuous blood glucose measurement, it is determined whether or not a next continuous blood glucose measurement should be performed, and output information including the determination is generated.
34. The method of item 28, wherein by considering the GA level acquired substantially simultaneously with the continuous blood glucose measurement, it is determined whether or not a next continuous blood glucose measurement should be performed.
35. The method of item 34, wherein when a rate of change of the GA level acquired after the continuous blood glucose measurement is greater than a predetermined value, output information indicating that the next continuous blood glucose measurement should be performed is generated.
36. The method of any one of items 33 to 35, further comprising generating a prescription of a drug or generating output information of an administration proposal in accordance with AGP after the continuous blood glucose measurement.
37. The method of any one of items 14 to 36, further comprising: referring to the prescription and/or medication information in the second data, and generating as output information a proposal for the next prescription and/or the administration, in accordance with the GA level or a trend of the GA levels, a value associated therewith or a combination thereof.
38. The method of item 37, further comprising referring to a medical history within the second data.
39. The method of item 38, wherein said generating as the output information comprises at least one of:
   if the medication information of second data of a type 2 diabetic patient includes a history of receiving sulfonylurea, a type of oral hypoglycemic agent, proposing candidates in the order of α-GI, a DPP-4 inhibitor (alert to hypoglycemia), and an SGLT2 inhibitor, when the patient's GA level is determined to be above the limit and postprandial blood glucose level is high;
   if the medication information of second data of a type 2 diabetic patient includes a history of receiving sulfonylurea, a type of oral hypoglycemic agent, proposing prescription drugs to be safely and inexpensively added in the order of a biguanide drug, a DPP-4 inhibitor, when the patient's GA level is above the limit and the fasting glucose level is high;
   if the medication information of second data of a type 2 diabetic patient includes a history of administration of a biguanide drug, proposing additional drugs: in the order of α-GI, a glinide drug, a DPP-4 inhibitor, and an SGLT2 inhibitor when the GA limit value
      determined by referring to the second data is exceeded and the postprandial blood glucose level is high, and in the order of a thiazoline drug, a DPP-4 inhibitor (alert to hypoglycemia), and an SGLT2 inhibitor when the fasting blood glucose level is high;
   if the medication information of second data of a type 2 diabetic patient includes a history of administration of a thiazoline drug, proposing to administer in the order of α-GI, a glinide drug when the GA limit value determined by referring to the second data is exceeded and the postprandial blood glucose level is high;
   if the medication information of second data of a type 2 diabetic patient includes a history of administration of a thiazoline drug, proposing to administer a biguanide drug as an additional drug when the GA limit value determined by referring to the second data is exceeded and the fasting blood glucose level is high;
   if the medication information of second data of a type 2 diabetic patient includes a history of administration of a DPP-4 inhibitor, proposing to administer in the order of α-GI, a glinide drug, when the GA limit value determined by referring to the second data is exceeded and the postprandial blood glucose level is high;
   if the medication information of second data of a type 2 diabetic patient includes a history of administration of a DPP-4 inhibitor, proposing to administer additional drugs in the order of a biguanide drug, a thiazoline drug, or an SGLT2 inhibitor when the GA limit value determined by referring to the second data is exceeded and the fasting blood glucose level is high;
   if the medication information of second data of a type 2 diabetic patient includes a history of administration of an SGLT2 inhibitor, proposing to administer α-GI when the GA limit value determined by referring to the second data is exceeded and the postprandial blood glucose level is high;
   if the medication information of second data of a type 2 diabetic patient includes a history of administration of an SGLT2 inhibitor, proposing to administer additional drugs in the order of a biguanide drug, a DPP-4 inhibitor, when the GA limit value determined by referring to the second data is exceeded and the fasting blood glucose level is high;
   if the medication information of second data of a type 2 diabetic patient includes a history of administration of a GLP-1 receptor agonist, proposing to administer α-GI when the GA limit value determined by referring to the second data is exceeded and the postprandial blood glucose level is high; and
   if the medication information of second data of a type 2 diabetic patient includes a history of administration of a GLP-1 receptor agonist, proposing to administer additional drugs in the order of a biguanide drug and an SGLT2 inhibitor when the GA limit value determined by referring to the second data is exceeded and the fasting blood glucose level is high.
40. The method of item 38 or 39, wherein said generating as the output information comprises at least one of:
   in the case of a type 1 or type 2 diabetic patient,
   proposing to administer α-GI, a glinide drug, to patients who are receiving an injections a day of persistent lytic insulin (glargine or detemir) at dinner or before going to bed, in addition to oral hypoglycemic agents, when the GA level exceeds the GA limit value determined by referring to the second data, and the postprandial blood glucose level is high;
   proposing to administer additional drugs in the order of a biguanide drug, a DPP-4 inhibitor, to patients who are receiving an injections a day of persistent lytic insulin (glargine or detemir) at dinner or before going to bed, in addition to oral hypoglycemic agents, when the GA level exceeds the GA limit value determined by referring to the second data, and the fasting blood glucose level is high;
   proposing to administer α-GI, a glinide drug, to patients who are receiving one injections per day of persistent lytic insulin (glargine or detemir) in the morning, in addition to oral hypoglycemic agents, when the GA level exceeds the GA limit value determined by referring to the second data, and the postprandial blood glucose level is high;
   proposed to administer in the order of a biguanide drug, a DPP-4 inhibitor, to patients who are receiving an injections a day of persistent lytic insulin (glargine or detemir) in the morning, in addition to oral hypoglycemic agents, when the GA level exceeds the GA limit value determined by referring to the second data, and the fasting blood glucose level is high;
      and
   proposing to administer a DPP-4 inhibitor to patients who are receiving an injections a day of persistent lytic insulin (glargine or detemir) in the morning, in addition to oral hypoglycemic agents, when the GA level exceeds the GA limit value determined by referring to the second data, and the fasting and postprandial blood glucose levels are both high.
41. The method of item 20, wherein a suggestion regarding a lifestyle habit or a treatment is generated as output information, by referring to the first data and the mean value and the variation range of the glucose levels of the continuous blood glucose measurement.
42. The method of item 41, further comprising providing output information including a suggestion regarding the lifestyle habit or the treatment to a diabetic patient, a prediabetic or a healthy person.
43. The method of item 20, wherein by referring to the first data and the mean value and the variation range of the glucose levels of the continuous blood glucose measurement, a diabetes mellitus risk % or an estimated time period until diabetes mellitus is contracted is generated as output information.
44. The method of item 43, further comprising providing output information including the diabetes mellitus risk % or the estimated time period until diabetes is contracted, to a prediabetic or a healthy person.
45. The method of item 20, wherein by referring to the first data and the mean value and the variation range of the glucose levels of the continuous blood glucose measurement, a diabetic complication risk % or an estimated time period until a complication is contracted is generated as output information.
46. The method of item 45, further comprising providing output information including the diabetic complication risk % or the estimated time period until a complication is contracted, to a diabetic patient.
47. The method of any one of items 11 to 46, further comprising notifying a collector of a body fluid that it should be collected.
48. The method of item 47, wherein said notifying comprises notifying that the body fluid should be collected at a frequency of substantially once every 17 days or 2 weeks.
49. The method of item 17 or 48, wherein said notifying comprises notifying that the timing of collection of the body fluid is approaching, that it is the timing, or that the timing is passed.
50. The method of any one of items 47 to 49, wherein said notifying comprises notifying the action to be taken related to a collection or a measurement of the body fluid.
51. The method of any one of items 11 to 46, further comprising searching for a correlation between the first data and the second data and generating third data comprising the correlation as an attribute.
52. The method of item 48, wherein the output information includes the third data.
53. The method of any one of items 1 to 52, wherein the body fluid is blood, tears or saliva.
54. A software for executing methods of items 1 to 53.
55. A storage medium storing a software for performing the method of items 1 to 53.
56. A healthcare management system, the system being configured to be connected to a sensor for measuring a GA level in a body fluid, and
   the system comprising:
   a memory configured to store:
      first data including a glycated albumin concentration and an albumin concentration in a plurality of body fluids, measured by the sensor, and a measurement time thereof, and
      second data including at least one of personal data, health-related data, lifestyle habit data, and population data;
   an analysis center configured to analyze the first data and the second data; and
   a computer configured to provide useful healthcare-related information to a user based on the analysis results by the analysis center.
57. A data structure for healthcare management, comprising:
   a first data array configured to allow addition of first data including a glycated albumin concentration and an albumin concentration in a body fluid measured by a glycated albumin sensor and their measurement times; and
   a second data array comprising at least one of personal data, health-related data, lifestyle habit data, and population data, and

   the data structure being configured to, by a computer,
      refer to the first data and the second data,
      generate output information including at least one of:
         a GA level, a converted HbA1c level, a comparison with a previous value, a mean blood glucose level;
   estimates of these values during one or more measurement periods in the past;
      predicted values for one or more future measurement periods;
      trend information of the past lifestyle habits;
      a risk of developing future diabetes or its complications and other diseases; and
      a recommendation on lifestyle habits,
   and provide the output information to a user.
58. The data structure for healthcare management of item 57, further comprising a third data array including third data including association information between the first data and the second data.

100 Medical information or healthcare management system
110 Communication network
121,122 Subject
131,132 GA measuring instrument
141,142 Smart devices
171,172 Non-subject user
150 Memory
160 AI analysis center
230 GA measurement device
231 GA measuring device body
232 Process part/arithmetic circuit block
233 Memory
234 Power supply
235 Display part
236 Communication part
237 Sensor
300 First data structure
310 Subject identification information
311 Name of subject
312 Subject ID
320 Measurement-related information
321 Measurement number
322 Sensor number (sensor management information)
323 Measurement time (measurement time information)
324 Measurement validity/invalidity information
325 Glycated albumin concentration
326 Albumin concentration
327 Other association information

## Claims

1. A medical information or healthcare management system (100) associated with glycated albumin (GA) levels, comprising:
a communication network (110);
a GA measuring device (131, 132), wherein the GA measuring device (131, 132) is used by a subject (121, 122) to measure their own body fluid;
a smart device (141, 142) connected to the GA measuring device (131, 132), the smart device (141, 142) being used as a display device or a communication device;
a memory (150) configured to store second data,
wherein GA levels obtained by measurements using the GA measuring device (131, 132) and time information of measurement times are transmitted as first data from the GA measuring device (131, 132) or the smart device (141, 142) to the memory (150) through the communication network (110) and stored;
an AI analysis center (160), wherein each configuration of the system (100) is connected to the AI analysis center (160) via the communication network (110),
wherein the AI analysis center (160) is configured to access the memory (150), analyze the first data and the second data, and generate analysis results as output information, and
wherein the output information is transmitted to the smart device (141, 142) of the subject (121, 122) via the communication network (110), thereby providing the output information to the subject (121, 122).

2. The system according to claim 1, wherein the body fluid is saliva.

3. The system according to claim 1 or 2, wherein the GA measuring device (131, 132) comprises a sensor (237) and is configured to measure the glycated albumin concentration in the body fluid non-invasively.

4. The system according to claim 3, wherein the sensor (237) is a glycated protein sensor comprising an immobilized protease, an immobilized ketoamine oxidase and a hydrogen peroxide detector.

5. The system according to claim 3 or 4, wherein the GA measuring device (131, 132) comprises a main body (231) in which a process unit (232), a memory (233), a power supply (234), a display unit (235) and a communication unit (236) are disposed and connected to each other, the sensor (237) being interchangeably or removably mounted to the main body (231), and wherein the process unit (232) is configured to control the sensor (237), to receive an output signal therefrom, to perform operations on that signal and to send the results to the display unit (235), the communication unit (236) and the memory (233).

6. The system according to claim 5, wherein the power supply (234) is a rechargeable or interchangeable battery.

7. The system according to any one of claims 3 to 6, wherein the sensor (237) is configured to acquire the time information of the measurement together with the measurement of the glycated albumin concentration or of the albumin concentration, and to manage the sensors used or to be used.

8. The system according to any one of the preceding claims, wherein the first data further comprises information (310) identifying the subject and, as information (320) relating to the measurement, a sensor number (322) identifying the sensor used for that measurement.

9. The system according to any one of the preceding claims, wherein the smart device (141, 142) is configured to display the GA levels and the transition tendency of the GA levels as measurement results.

10. The system according to any one of the preceding claims, wherein the AI analysis center (160) is configured to determine, based on a change in the GA level acquired after a continuous blood glucose measurement, whether or not a next continuous blood glucose measurement should be performed, the output information including the determination.

11. The system according to any one of the preceding claims, wherein the second data comprises at least one of personal data, health-related data, lifestyle habit data and population data, and the AI analysis center (160) is configured to perform AI analysis by machine learning on the first data and the second data stored in the memory (150).

12. The system (100) according to any one of the preceding claims, wherein the smart device (141, 142) is directly connected to the GA measuring device (131, 132), wirelessly or wiredly without passing through the communication network (110).
